# EUROPÄISCHE PATENTSCHRIFT

(11) **EP 2 266 483 B1**
(45) Veröffentlichungstag und Bekanntmachung des Hinweises auf die Patenterteilung: **04.06.2014**
(21) Anmeldenummer: 10167273.1
(22) Anmeldetag: 25.06.2010
(51) Int. Cl.: A61B 17/70, A61B 17/00, A61B 17/17, A61B 17/80, A61B 17/84, A61B 17/88

(54) **Chirurgische Knochenverankerungsvorrichtung und Wirbelsäulenfixationssystem**
Surgical bone anchoring device and spinal column fixation system
Dispositif d'ancrage osseux chirurgical et système de fixation de la colonne vertébrale

(30) Priorität: 26.06.2009 DE 102009032034
(43) Veröffentlichungstag der Anmeldung: 29.12.2010
(73) Patentinhaber: AESCULAP AG, 78532 Tuttlingen (DE)
(72) Erfinder: Lindner, Stephan, 78573 Wurmlingen (DE)
(74) Vertreter: Hoeger, Stellrecht & Partner Patentanwälte

(56) Entgegenhaltungen:
- WO-A1-94/26190
- WO-A2-2008/045719
- DE-A1-102007 042 953
- DE-U1-202007 012 646

## Beschreibung

Die vorliegende Erfindung betrifft eine chirurgische Knochenverankerungsvorrichtung für ein Wirbelsäulenfixationssystem, umfassend einen Verankerungsteil zum Verankern in oder an einem Knochen, einen Lagerteil zum Lagern mindestens eines Verbindungselements zum Festlegen an mindestens zwei Knochenverankerungsvorrichtungen des Wirbelsäulenfixationssystems und ein Fixierelement, wobei der Verankerungsteil und der Lagerteil aneinander gelagert, in einer Montagestellung relativ zueinander bewegbar und mit dem Fixierelement in einer Implantationsstellung relativ zueinander festlegbar sind, welches Fixierelement einstückig ausgebildet ist und einen proximalen Fixierelementabschnitt, einen distalen Fixierelementabschnitt und einen Sollbruchbereich umfasst, welcher Sollbruchbereich zwischen dem proximalen und dem distalen Fixierelementabschnitt ausgebildet ist, wobei am distalen Fixierelementabschnitt eine Werkzeugelementaufnahme vorgesehen ist zum kraftund/oder formschlüssigen in Eingriff Bringen mit einem Werkzeug zum Überführen der Knochenverankerungsvorrichtung von der Montagestellung in die Implantationsstellung.

Ferner betrifft die vorliegende Erfindung ein Wirbelsäulenfixationssystem, umfassend mindestens eine chirurgische Knochenverankerungsvorrichtung und mindestens eines Verbindungselements zum Festlegen an mindes-tens zwei Knochenverankerungsvorrichtungen des Wirbelsäulenfixationssystems, welche mindestens eine chirurgische Knochenverankerungsvorrichtung einen Verankerungsteil zum Verankern in oder an einem Knochen, einen Lagerteil zum Lagern mindestens eines Verbindungselements zum Festlegen an mindestens zwei Knochenverankerungsvorrichtungen des Wirbelsäulenfixationssystems und ein Fixierelement, wobei der Verankerungsteil und der Lagerteil aneinander gelagert, in einer Montagestellung relativ zueinander bewegbar und mit dem Fixierelement in einer Implantationsstellung relativ zueinander festlegbar sind, welches Fixierelement einstückig ausgebildet ist und einen proximalen Fixierelementabschnitt, einen distalen Fixierelementabschnitt und einen Sollbruchbereich umfasst, welcher Sollbruchbereich zwischen dem proximalen und dem distalen Fixierelementabschnitt ausgebildet ist, wobei am distalen Fixierelementabschnitt eine Werkzeugelementaufnahme vorgesehen ist zum kraft- und/oder formschlüssigen in Eingriff Bringen mit einem Werkzeug zum Überführen der Knochenverankerungsvorrichtung von der Montagestellung in die Implantationsstellung.

Chirurgische Knochenverankerungsvorrichtungen, ebenso wie ein Wirbelsäulenfixationssystem, sind beispielsweise aus der US 6,299,616 bekannt. Bei dem bekannten System ist es jedoch erforderlich, einen Drehmomentschlüssel einzusetzen, um das Fixierelement mit einem definierten Haltemoment gegen den Verankerungsteil zu spannen, um das Lagerteil unbeweglich am Verankerungsteil in der Implantationsstellung zu fixieren. Eine Weiterbildung derartiger Knochenverankerungsvorrichtungen ist aus der US 2009/0105718 A1 bekannt. Das darin offenbarte Fixierelement weist zwischen einem distalen und einem proximalen Fixierelementabschnitt einen Sollbruchbereich auf. Es dient dem Zweck, einen Drehmomentschlüssel überflüssig zu machen. Daher ist ausschließlich am proximalen Fixierelementabschnitt eine Werkzeugaufnahme vorgesehen für ein Einschraubwerkzeug. Wird beim Verspannen des Fixierelements gegen den Verankerungsteil das durch den Sollbruchbereich vorgegebene Bruchmoment überschritten, schert das Fixierelement zwischen dem distalen und dem proximalen Fixierelementabschnitt ab. Der distale Fixierelementabschnitt hält dann den Lagerteil mit einem Haltemoment, welches dem Abschermoment des Sollbruchbereichs entspricht, klemmend am Verankerungsteil.

Ausgehend von dem oben genannten Stand der Technik stellt sich jedoch bei einem chirurgischen Eingriff das Problem, dass das Fixierelement nur sehr umständlich an das Lagerteil heranführbar und mit diesem verbindbar ist, insbesondere verschraubbar, um den Lagerteil gegen den Verankerungsteil zu verspannen. Es ist bekannt, lange Führungshülsen mit dem Lagerteil temporär zu verbinden, um das Einführen des Fixierelements sowie hierfür benötigter Werkzeuge zu erleichtern. Das Verbinden von Führungshülsen mit dem Lagerteil ist jedoch nicht sehr einfach, insbesondere dann nicht, wenn die Sicht auf die miteinander zu verbindenden Teile erschwert ist und erfordert daher großes handwerkliches Geschick des Operateurs. Zudem kostet jeder OP-Schritt zusätzlich Zeit.

Eine Knochenverankerungsvorrichtung und ein orthopädisches Haltesystem sind in der DE 10 2007 042 953 A1 beschrieben. Ferner offenbart die WO 94/26190 ein osteosynthetisches Befestigungselement und eine zugehörige Manipulierhilfe. Aus der DE 20 2007 012 646 U1 sind eine Knochenverankerungsvorrichtung und ein Wirbelsäulenfixationssystem der eingangs beschriebenen Art bekannt.

Es ist daher eine Aufgabe der vorliegenden Erfindung, eine chirurgische Knochenverankerungsvorrichtung sowie ein Wirbelsäulenfixationssystem der eingangs beschriebenen Art so zu verbessern, dass ein chirurgischer Eingriff, insbesondere zur Stabilisierung einer Wirbelsäule, vereinfacht wird.

Diese Aufgabe wird sowohl bei einer chirurgischen Knochenverankerungsvorrichtung als auch bei einem Wirbelsäulenfixationssystem der eingangs beschriebenen Art erfindungsgemäß dadurch gelöst, dass die Knochenverankerungsvorrichtung ein Zentrierelement umfasst, welches mit dem Fixierelement am Lagerteil gehalten ist..

Die Weiterbildung bekannter Knochenverankerungsvorrichtungen und Wirbelsäulenfixationssysteme durch die vorgeschlagene Gestaltung des Fixierelements ermöglicht es, mittels des Fixierelements ein Zentrierelement vor dem Einbringen in einen Patientenkörper mit dem Lagerteil zu verbinden, also die Knochenverankerungsvorrichtung insgesamt so vorzumontieren, dass das Fixierelement bereits mit dem Lagerteil verbunden ist. Es erübrigt sich dann, das Fixierelement nach Festlegen des Verankerungsteils an einem Knochen und Ausrichten des Lagerteils relativ zum Verankerungsteil erst mit dem Lagerteil in Verbindung zu bringen. Dieser Montageschritt muss also nicht während des chirurgischen Eingriffs vorgenommen werden, sondern kann bereits bei der Montage der Knochenverankerungsvorrichtung erfolgen, insbesondere direkt bei der Herstellung derselben oder vor der Durchführung eines chirurgischen Eingriffs. Durch das Vorsehen der Werkzeugelementaufnahme vorzugsweise ausschließlich am distalen Fixierelementabschnitt, hat ein Operateur individuell die Möglichkeit, ein Haltemoment festzulegen, welches er beispielsweise mit einem dafür vorgesehenen Drehmomentschlüssel aufbringen kann. Des Weiteren kann, zum Beispiel durch Vorsehen eines mittels des Fixierelements am Lagerteil gehaltenen Zentrierelements, vermieden werden, dass nach Zerstören des Sollbruchbereichs der proximale Fixierelementabschnitt im oder am Operationssitus verloren gehen kann. Es ist dann insbesondere denkbar den proximalen Fixierelementabschnitt zusammen mit dem Zentrierelement vom Operationssitus zu entfernen. Das Zentrierelement ermöglicht es, weitere Instrumente und gegebenenfalls Halte-, Klemm- oder Spannelemente der Knochenverankerungsvorrichtung beziehungsweise des Wirbelsäulenfixationssystems einfach und sicher an die Knochenverankerungsvorrichtung heranzuführen. Ein kompliziertes Verbinden und gegebenenfalls Lösen des Zentrierelements während des chirurgischen Eingriffs mit dem Lagerteil entfällt, wodurch sich eine Operationszeit deutlich verkürzen lässt. Ferner kann beispielsweise auch auf eine Verschraubung des Zentrierelements mit dem Lagerteil verzichtet werden, wenn das Fixierelement das Zentrierelement entsprechend gegen das Lagerteil spannt oder hält. Dies ermöglicht ferner eine Vormontage der Verankerungseinrichtung derart, dass das zum Festlegen des Lagerteils am Verankerungsteil vorgesehene Fixierelement bereits an der Knochenverankerungsvorrichtung vormontiert werden kann und gleichzeitig den Zweck erfüllt, das Zentrierelement temporär am Lagerteil zu halten. Ferner besteht so auch die Möglichkeit, das Zentrierelement und das Fixierelement so auszubilden, dass der Sollbruchbereich trennbar ist durch Beaufschlagen des Fixierelements, insbesondere dessen distalen Fixierelementsabschnitt, mit einem Zug- und Drehmoment. Vorzugsweise ist das Zentrierelement durch das Fixierelement unbeweglich am Lagerteil gehalten.

Besonders einfach wird der Aufbau des Zentrierelements, wenn es in Form einer Zentrierhülse ausgebildet ist. Die Zentrierhülse ermöglicht es, Teile durch sie hindurch und außen über sie an die Knochenverankerungsvorrichtung, insbesondere deren Lagerteil, heranzuführen.

Vorteilhafterweise umfasst die chirurgische Knochenverankerungsvorrichtung eine Positioniereinrichtung zum Positionieren des Zentrierelements und des Lagerteils relativ zueinander in der Montagestellung. Insbesondere ermöglicht es die Positioniereinrichtung, das Zentrierelement in definierter Weise mittels des Fixierelements am Lagerteil zu halten.

Besonders einfach wird der Aufbau der Positioniereinrichtung, wenn diese erste und zweite Positionierglieder umfasst, welche einerseits am Zentrierelement und andererseits am Lagerteil angeordnet oder ausgebildet sind, und wenn die ersten und zweiten Positionierglieder in der Montagestellung kraft- und/oder formschlüssig miteinander in Eingriff stehen. Insbesondere kann die Positioniereinrichtung derart ausgebildet sein, dass die ersten und zweiten Positionierglieder eine Verdrehsicherung ausbilden, so dass verhindert wird, dass das Zentrierelement beispielsweise um eine von ihm definierte Längsachse relativ zum Lagerteil verdrehbar ist. Dies gestattet es, das Zentrierelement mit dem Fixierelement in definierter Weise am Lagerteil zu halten, beispielsweise das Zentrierelement gegen das Lagerteil zu spannen. Abgesehen von der Positioniereinrichtung sind dann keine weiteren Verbindungselemente erforderlich, um das Zentrierelement am Lagerteil zu halten. Hierfür kann insbesondere ausschließlich das Fixierelement genutzt werden.

Der Aufbau der Positioniereinrichtung lässt sich weiter vereinfachen, wenn ein erstes Positionierglied in Form eines Positioniervorsprungs und wenn ein zweites Positionierglied in Form einer zum Positioniervorsprung korrespondierenden Positionieraufnahme ausgebildet ist. Der Positioniervorsprung und die Positionieraufnahme können wahlweise am Zentrierelement oder am Lagerteil angeordnet oder ausgebildet sein.

Auf einfache Weise in Eingriff bringen lassen sich die ersten und zweiten Positionierglieder, wenn sie in eine Richtung parallel oder im Wesentlichen parallel zu einer Längsachse des Zentrierelements weisen.

Günstigerweise hält das Fixierelement das Zentrierelement in der Montagestellung unbeweglich am Lagerteil. Dies gestattet eine besonders gute Führung weiterer Implantatteile oder von Instrumenten eines für die Implantation verwendeten Instrumentariums mittels des Zentrierelements.

Vorteilhaft ist, wenn das Fixierelement das Zentrierelement in der Montagestellung kraft- und/oder formschlüssig am Lagerteil hält. Besonders einfach lässt sich das Zentrierelement am Lagerteil halten, wenn das Fixierelement das Zentrierelement in der Montagestellung gegen das Lagerteil spannt.

Günstig kann es ferner sein, wenn das Fixierelement das Zentrierelement in der Montagestellung klemmend am Lagerteil hält. Insbesondere ist es so möglich, dass ein Teil oder ein Abschnitt des Zentrierelements in der Montagestellung klemmend zwischen einem entsprechenden Abschnitt des Fixierelements und dem Lagerteil haltbar ist.

Gemäß einer weiteren bevorzugten Ausführungsform der Erfindung kann ferner eine Kopplungseinrichtung zum Koppeln des Zentrierelements und des Fixierelements vorgesehen sein. Die Kopplungseinrichtung kann insbesondere eine bewegliche oder unbewegliche Kopplung des Zentrierelements und des Fixierelements relativ zueinander ermöglichen. Ferner hat die Kopplungseinrichtung auch den Vorteil, dass nach Zerstören des Sollbruchbereichs insbesondere der proximale Fixierelementabschnitt und das Zentrierelement weiter miteinander gekoppelt sein können, und zwar derart, dass eine Relativbewegung zwischen dem Zentrierelement und dem proximalen Fixierelementabschnitt ermöglicht werden kann oder auch nicht.

Vorzugsweise umfasst die Kopplungseinrichtung erste und zweite Kopplungsglieder, welcher einerseits am Zentrierelement und andererseits am Fixierelement angeordnet sind, wobei die ersten und zweiten Kopplungsglieder in einer Kopplungsstellung miteinander in Eingriff stehen, in welcher die Knochenverankerungsvorrichtung die Montagestellung einnimmt. Miteinander in Eingriff stehen kann insbesondere auch bedeuten, dass die Kopplungsglieder aneinander anliegen und so eine Kopplung zwischen dem Zentrierelement und dem Fixierelement bewirken. Insbesondere können die Kopplungsglieder in Form von Anschlägen ausgebildet sein, die eine Relativbewegung des Zentrierelements und des Fixierelements parallel oder im Wesentlichen parallel zu einer vom Zentrierelement definierten Längsachse einschränken. Insbesondere kann am Fixierelement ein Anschlag vorgesehen sein, welcher eine Bewegung des Zentrierelements in proximaler Richtung vom Fixierelement weg, und optional auch vom Lagerteil weg, verhindert.

Besonders einfach wird die Ausbildung der Kopplungseinrichtung, wenn ein erstes Kopplungsglied in Form einer am Zentrierelement in proximaler Richtung weisenden ersten Anschlagfläche ausgebildet ist, wenn ein korrespondierendes zweites Kopplungsglied in Form einer am Fixierelement in distaler Richtung weisenden zweiten Anschlagfläche ausgebildet ist und wenn die ersten und zweiten Anschlagflächen in der Kopplungsstellung aneinander anliegen. Eine solche Kopplungseinrichtung ermöglicht es, mit dem Fixierelement das Zentrierelement gegen das Lagerteil zu spannen, insbesondere ohne dass das Zentrierelement relativ zum Lagerteil rotiert werden muss. Es kann so insbesondere auf eine Verschraubung des Zentrierelements mit dem Lagerteil verzichtet werden, was besonders schlanke Bauformen sowohl des Lagerteils als auch des Zentrierelements ermöglicht. Insbesondere ist es möglich, mit dem Fixierelement das Zentrierelement gegen das Lagerteil zu ziehen und spannend zu halten. Des Weiteren bilden die Kopplungsglieder optional auch Widerlager, die genutzt werden können, um die zum Zerstören des Sollbruchbereichs erforderlichen Zug- und/oder Torsionskräfte gegenzuhalten. Wird beispielsweise das Fixierelement ausgehend von der Montagestellung, in der es das Zentrierelement am Lagerteil hält, weiter in distaler Richtung bewegt, so können die Kopplungsglieder die zusätzlich auftretenden Torsions- und Zugkräfte aufnehmen. Überschreiten diese jedoch die Bruchwerte des Sollbruchbereichs, wird dieser zerstört und der distale Fixierelementabschnitt vom proximalen Fixierelementabschnitt getrennt. Der proximale Fixierelementabschnitt kann dann, da er noch mit dem Zentrierelement mittels der Kopplungseinrichtung gekoppelt ist, einfach und sicher aus dem Operationsgebiet entfernt werden. Der distale Fixierelementabschnitt kann dann genutzt werden, um die Knochenverankerungsvorrichtung in die Implantationsstellung überzuführen, in der der Lagerteil und der Verankerungsteil relativ zueinander nicht mehr beweglich sind.

Günstigerweise sichern die Positioniereinrichtung und die Kopplungseinrichtung das Zentrierelement in der Montagestellung und in der Kopplungsstellung am Lagerteil um eine vom Zentrierelement definierte Längsachse unverdrehbar. Dies gestattet eine definierte Positionierung des Zentrierelements und des Lagerteils relativ zueinander.

Gemäß einer weiteren bevorzugten Ausführungsform der Erfindung kann vorgesehen sein, dass das Fixierelement eine Fixierfläche aufweist, welche in der Implantationsstellung an einem Fixierflächenbereich des Verankerungsteils anliegt, und dass die Fixierfläche vom Fixierflächenbereich beabstandet ist, wenn die Kopplungseinrichtung die Kopplungsstellung einnimmt und solange der distale und der proximale Fixierelementabschnitt über den Sollbruchbereich miteinander verbunden sind. Diese Ausbildung der Knochenverankerungsvorrichtung stellt sicher, dass der Lagerteil und der Verankerungsteil erst dann unbeweglich aneinander festgelegt werden können, wenn der Sollbruchbereich zerstört ist. Solange das Fixierelement unbeschädigt ist, ist es also nicht möglich, die Fixierfläche an den Fixierflächenbereich heranzuführen. Hierfür ist, aufgrund der besonderen Ausbildung und Anordnung der Kopplungseinrichtung, das Zerstören des Sollbruchbereichs erforderlich. Soll also die Knochenverankerungsvorrichtung in die Implantationsstellung überführt werden, muss der Sollbruchbereich zerstört werden, wodurch die beiden Fixierelementabschnitte voneinander getrennt werden. Der distale Fixierelementabschnitt dient dann zum unbeweglichen Festlegen des Lagerteils am Verankerungsteil und der zum Halten des Zentrierelements am Lagerteil genutzte proximale Fixierelementabschnitt kann einfach und sicher mit Hilfe des Zentrierelements vom Operationsgebiet entfernt werden. Es ist mit der verbesserten Knochenverankerungsvorrichtung also möglich, in einem einzigen Operationsschritt den Lagerteil und den Verankerungsteil relativ zueinander unbeweglich festzulegen und das Zentrierelement vom Lagerteil abzutrennen und zu entfernen.

Vorteilhaft ist es, wenn der Lagerteil und der Verankerungsteil relativ zueinander beweglich sind, wenn der Sollbruchbereich unbeschädigt ist. Mit anderen

Worten bedeutet dies, dass, solange das Zentrierelement mittels des Fixierelements am Lagerteil gehalten ist, der Lagerteil und der Verankerungsteil relativ zueinander bewegbar und somit justierbar sind. Erst dann, wenn alle Teile der Knochenverankerungsvorrichtung beziehungsweise des Wirbelsäulenfixationssystems im Körper angeordnet und festgelegt sind, kann die vom Operateur vorgesehene Relativstellung des Lagerteils und des Verankerungsteils dauerhaft fixiert, mittels des Fixierelements, und das Zentrierelement in der beschriebenen Weise entfernt werden.

Um sicher zu stellen, dass einmal verwendete Fixierelemente nicht mehrfach verwendet werden können, ist es günstig, wenn der distale und der proximale Fixierelementabschnitt irreversibel voneinander trennbar sind durch Zerstören des Sollbruchbereichs.

Gemäß einer weiteren bevorzugten Ausführungsform der Erfindung kann vorgesehen sein, dass der Sollbruchbereich durch eine Schwächung des Fixierelements zwischen dem distalen und dem proximalen Fixierelementabschnitt ausgebildet ist. Beispielsweise kann eine Wandstärke des Fixierelements zur Ausbildung des Sollbruchbereichs verringert sein. Herstellen lässt sich dies beispielsweise durch einen Einstich in einer äußeren Wandfläche eines hülsenförmigen Fixierelements.

Besonders einfach lässt sich das Fixierelement am Lagerteil festlegen, wenn diese miteinander verschraubbar ausgebildet sind. Zudem können so auch die zur dauerhaften Klemmung des Lagerteils und des Verankerungsteils erforderlichen Klemmkräfte sicher aufgebracht werden.

Vorteilhaft ist es, wenn das Fixierelement einen Außengewindeabschnitt aufweist und wenn der Lagerteil einen korrespondierenden Innengewindeabschnitt aufweist. Dies gestattet es, das Fixierelement im Inneren beispielsweise eines hülsenförmigen Abschnitts des Lagerteils einzuschrauben und ein darin angeordnetes Kopfteil eines Verankerungselements klemmend in der Implantationsstellung zu sichern.

Grundsätzlich wäre es zwar denkbar, den Außengewindeabschnitt am proximalen Fixierelementabschnitt auszubilden. Günstiger ist es jedoch, wenn der Außengewindeabschnitt am distalen Fixierelementabschnitt ausgebildet ist. Im Zusammenwirken mit einem Einschraubwerkzeug, welches in die am distalen Fixierelementabschnitt ausgebildete Werkzeugelementaufnahme eingreift, kann so der distale Fixierelementabschnitt in gewünschter Weise genutzt werden, nämlich wie eine herkömmliche Fixierschraube, um am Ende des chirurgischen Eingriffs gegen zum Beispiel einen am oder im Lagerteil gehaltenen Kopfteil des Verankerungsteils gespannt zu werden.

Besonders kompakt ausbilden lässt sich das Fixierelement, wenn der Sollbruchbereich proximalseitig an den Außengewindeabschnitt angrenzt. So kann insbesondere sichergestellt werden, dass nur der Abschnitt des Fixierelements, der tatsächlich zum Fixieren der Knochenverankerungsvorrichtung in der Implantationsstellung dient, letztendlich im Körper des Patienten verbleibt.

Um die Montage des Zentrierelements mittels des Fixierelements am Lagerteil zu erleichtern, ist es günstig, wenn der proximale Fixierelementabschnitt einen größeren Außendurchmesser aufweist als der distale Fixierelementabschnitt.
Dies ermöglicht es insbesondere, den distalen Fixierelementabschnitt von proximal her kommend durch das Zentrierelement durchzuführen derart, dass der distale Fixierelementabschnitt distalseitig über das Zentrierelement vorsteht und mit dem Lagerteil in Eingriff bringbar ist, insbesondere verschraubbar.

Vorzugsweise ist das Fixierelement hülsenförmig ausgebildet. Die hülsenförmige Ausgestaltung ermöglicht es, beispielsweise mit einem Werkzeug durch das Fixierelement hindurch auf ein proximales Ende des Verankerungsteils zuzugreifen, um beispielsweise das Verankerungsteil weiter in einen Knochen einzutreiben, zum Beispiel einzuschrauben, oder auch wieder aus diesem heraus zu bewegen.

Vorteilhafterweise ist die Werkzeugelementaufnahme in Form eines Innenvielkant oder Innenvielrund ausgebildet. So können üblicherweise vorhandene Einschraubwerkzeuge, die in Form eines Außenvielkant oder eines Außenvielrund ausgebildet sind, zum in Eingriff bringen mit dem Fixierelement genutzt werden.

Damit der Verankerungsteil auf einfache Weise in einen Knochen eingebracht, beispielsweise eingeschraubt werden kann, ist es vorteilhaft, wenn der Verankerungsteil eine Werkzeugaufnahme aufweist, welche in proximaler Richtung weisend geöffnet ist.

Herkömmliche Einschraubwerkzeuge lassen sich zum Einbringen des Verankerungsteils in einen Knochen nutzen, wenn die Werkzeugaufnahme in Form eines Innenvielkant oder eines Innenvielrund ausgebildet ist.

Gemäß einer weiteren bevorzugten Ausführungsform der Erfindung kann die Knochenverankerungsvorrichtung eine Klemmeinrichtung umfassen zum klemmenden Halten eines Verbindungselements des Wirbelsäulenfixationssystems am Lagerteil. Das Verbindungselement kann insbesondere an zwei Knochenverankerungsvorrichtungen fixiert werden, um beispielsweise zwei Wirbelkörper in einem vom Operateur gewünschten Abstand voneinander zu fixieren.

Besonders einfach handhabbar wird die Knochenverankerungsvorrichtung, wenn die Klemmeinrichtung mindestens eine Verbindungselementaufnahme zum Aufnehmen eines Verbindungselements oder eines Teils desselben und ein Außengewinde am Lagerteil und eine Mutter mit einem zum Außengewinde korrespondierenden Innengewinde umfasst, welche Mutter eine die Verbindungselementaufnahme quer zu einer von dieser definierten Längsachse eröffnende Einführöffnung in einer Klemmstellung mindestens teilweise verkleinert. Mit einer solchen Klemmeinrichtung kann ein mindestens teil- oder abschnittsweise in die Verbindungselementaufnahme eingeführtes Verbindungselement mittels der Mutter in der Klemmstellung klemmend gehalten werden. Um eine Klemmung des Verbindungselements in der Verbindungselementaufnahme zu optimieren, ist es vorteilhaft, wenn eine Längsachse der Verbindungselementaufnahme und eine Längsachse des Zentrierelements in der Montagestellung windschief zueinander verlaufen. Windschief kann insbesondere bedeuten, dass Ebenen, die senkrecht zu den beiden Längsachsen verlaufen, sich senkrecht schneiden und insbesondere die jeweils andere Längsachse enthalten oder zumindest parallel zu dieser verlaufen.

Vorteilhaft kann es ferner sein, wenn die Knochenverankerungsvorrichtung eine Sicherungseinrichtung umfasst zum Sichern des proximalen Fixierelementabschnitts am Zentrierelement nach dem Trennen der distalen und proximalen Fixierelementabschnitte voneinander. Mit der Sicherungseinrichtung kann insbesondere verhindert werden, dass sich der vom distalen Fixierelementabschnitt abgetrennte proximale Fixierelementabschnitt vom Zentrierelement in unbeabsichtigter Weise lösen und verloren gehen kann.

Besonders einfach wird der Aufbau der Sicherungseinrichtung, wenn diese eine Innenverzahnung am Zentrierelement proximalseitig des am Zentrierelement vorgesehen Kopplungsglieds und eine korrespondierende Außenverzahnung des proximalen Fixierelementabschnitts umfasst. Dies ermöglicht es, den proximalen Fixierelementabschnitt von proximal her kommend in das Zentrierelement einzuführen, wobei die Verzahnungen beim Montieren des Fixierelements am Zentrierelement über eine von ihnen definierte Länge lediglich eine Bewegung des Fixierelements parallel zur Längsachse des Zentrierelements ermöglichen und erst dann wieder eine Rotation um die Längsachse, wenn die Verzahnungen außer Eingriff stehen. Dabei genügt dann schon eine geringe Rotation um eine Bewegung des Fixierelements wieder in proximaler Richtung zu verhindern. Die Innenverzahnung des Zentrierelements bildet dann einen Anschlag für den proximalen Fixierelementabschnitt, wodurch eine Bewegung des Fixierelements in proximaler Richtung verhindert wird. Insbesondere kann so der proximale Fixierelementabschnitt zwischen der Innenverzahnung und einem der beiden Kopplungsglieder der Kopplungseinrichtung, welches am Zentrierelement abgeordnet ist, gesichert werden, um ein Lösen des proximalen Fixierelementabschnitts vom Zentrierelement zu verhindern.

Um das Einführen des Fixierelements von proximal her durch das Zentrierelement zu ermöglichen, ist es vorteilhaft, wenn ein maximaler Innendurchmesser der Innenverzahnung im Bereich eines Zahngrunds gleich oder größer als ein maximaler Außendurchmesser der Außenverzahnung ist.

Zur Ausbildung eines Anschlags zum Verhindern einer Bewegung des Fixierelements in proximaler Richtung ist es vorteilhaft, wenn ein minimaler Innendurchmesser der Innenverzahnung kleiner ist als ein maximaler Außendurchmesser der Außenverzahnung.

Gemäß einer weiteren bevorzugten Ausführungsform der Erfindung kann vorgesehen sein, dass der Lagerteil mindestens eine Befestigungselementaufnahme für ein an einem Knochen festlegbares Befestigungselement umfasst. Insbesondere kann das Befestigungselement zusätzlich zum Verankerungsteil vorgesehen sein, welcher ebenfalls ein Befestigungselement in diesem Sinne bildet.

Vorzugsweise sind der Lagerteil und der Verankerungsteil in der Montagestellung relativ zueinander verschwenkbar. So lassen sich von einem Operateur gewünschte Winkel zwischen Längsachsen des Lagerteils und des Verankerungsteils während des chirurgischen Eingriffs einstellen und gegebenenfalls mittels des Fixierelements fixieren.

Besonders flexibel bei der Justage ist ein Operateur, wenn der Lagerteil und der Verankerungsteil in der Montagestellung kugelgelenkig aneinander gelagert sind. Beispielsweise kann zur Ausbildung eines Kugelgelenks der Verankerungsteil ein kugeliges proximales Ende aufweisen, welches korrespondierend zu einem hohlkalottenförmigen, am Lagerteil vorgesehenen Sitz ausgebildet ist.

Ganz besonders einfach und sicher lassen sich der Lagerteil und der Verankerungsteil in der Implantationsstellung aneinander festlegen, wenn das Fixierelement in Form eines Klemmelements ausgebildet ist zum klemmenden Halten eines Kopfabschnitts des Verankerungsteils in einem Sitz des Lagerteils für den Kopfabschnitt.

Einfach und sicher lässt sich die Knochenverankerungsvorrichtung an einem Knochen verankern, wenn der Verankerungsteil in Form eines Schraubenkörpers mit einem Außengewinde ausgebildet ist. Das Außengewinde ist vorzugsweise in Form eines Knochengewindes ausgebildet.

Alternativ ist es selbstverständlich auch denkbar, den Verankerungsteil in Form eines Hakens auszubilden, insbesondere in Form eines Knochenhakens, der in einen Knochen eingeschlagen werden kann.

Günstig ist es, wenn mindestens eine Knochenverankerungsvorrichtung des Wirbelsäulenfixationssystems in Form einer der oben beschriebenen Knochenverankerungsvorrichtungen ausgebildet ist. Das Wirbelsäulenfixationssystem weist dann als Ganzes die bereits oben im Zusammenhang mit bevorzugten Ausführungsformen der Knochenverankerungsvorrichtungen beschriebenen Vorteile auf.

Günstigerweise umfasst das Wirbelsäulenfixationssystem mindestens zwei Knochenverankerungsvorrichtungen. Diese lassen sich insbesondere an zwei benachbarten Wirbeln festlegen, so dass mittels eines an den Knochenverankerungsvorrichtungen festlegbaren Verbindungselements die beiden Wirbelkörper in einem definierten Abstand voneinander fixiert werden können.

Vorteilhaft ist es, wenn das Wirbelsäulenfixationssystem mindestens ein an zwei Knochenverankerungseinrichtungen festlegbares Verbindungselement umfasst. So können in der beschriebenen Weise beispielsweise zwei Wirbelkörper mittels des Wirbelsäulenfixationssystems in einem vordefinierten Abstand voneinander festgelegt werden.

Besonders einfach wird der Aufbau des Wirbelsäulenfixationssystems, wenn das mindestens eine Verbindungselement stabförmig ausgebildet ist oder einen stabförmigen oder einen ringförmigen Verbindungsabschnitt aufweist. Insbesondere ist das Verbindungselement vorzugsweise derart ausgebildet, dass es teilweise in eine entsprechende Verbindungselementaufnahme an der Knochenverankerungsvorrichtung, insbesondere am Lagerteil, eingebracht und mittels einer dafür vorgesehenen Klemmvorrichtung klemmend fixiert werden kann.

Die nachfolgende Beschreibung bevorzugter Ausführungsformen der Erfindung dient im Zusammenhang mit den Zeichnungen der näheren Erläuterung. Es zeigen:
- Figur 1:: eine teilweise Schnittansicht durch ein K-Drahtapplikationsinstrument, jedoch nicht erfindungsgemäß;
- Figur 2:: eine teilweise durchbrochene Seitenansicht beim Applizieren des K-Drahts in einen Wirbelknochen mit Hilfe des in Figur 1 dargestellten Instruments;
- Figur 3:: eine schematische Darstellung eines Teils einer Knochenverankerungsvorrichtung beim Montieren von deren Lagerteil und Verankerungsteil aneinander;
- Figur 4:: eine perspektivische Ansicht der Knochenverankerungsvorrichtung aus Figur 3 beim Vormontieren eines Zentrierelements mittels eines Fixierelements;
- Figur 5:: eine ausschnittsweise, teilweise geschnittene Ansicht der vormontierten Knochenverankerungsvorrichtung aus Figur 4;
- Figur 6:: eine perspektivische, teilweise durchbrochene Ansicht der Knochenverankerungsvorrichtung aus Figur 5 vor dem Verbinden mit einem nicht erfindungsgemäßen Halte- und Einsetzinstrument;
- Figur 7:: eine teilweise geschnittene perspektivische Ansicht der Knochenverankerungsvorrichtung mit verbundenem Halte- und Einsetzinstrument;
- Figur 8:: eine perspektivische Gesamtansicht der Anordnung aus Figur 7 mit zusätzlichem, nicht erfindungsgemäßen, Einschraubwerkzeug für den Verankerungsteil der Knochenverankerungsvorrichtung beim Aufschieben auf den im Wirbelkörper eingesetzten K-Draht;
- Figur 9:: eine Längsschnittansicht eines distalen Endbereichs der in Figur 8 dargestellten Gesamtanordnung beim Einschrauben des Verankerungsteils;
- Figur 10:: eine Ansicht ähnlich Figur 8 nach Einschrauben des Verankerungsteils in den Wirbelkörper beim Herausziehen des K-Drahts aus dem Wirbelkörper mittels eines K-Drahtentfernungsinstruments;
- Figur 11:: eine perspektivische Gesamtansicht zweier schematisch dargestellter Wirbelkörper beim Verbinden derselben mittels einer Verbindungsplatte durch Aufsetzen derselben auf zwei in den Wirbelkörper verankerter Knochenverankerungsvorrichtungen;
- Figur 12:: eine perspektivische, teilweise durchbrochene Gesamtansicht des Halte- und Einsetzinstruments beim Einführen in eine Einschraubhülse mit darin gehaltener Befestigungsmutter;
- Figur 13:: eine perspektivische, teilweise durchbrochene Gesamtansicht der Anordnung aus Figur 12 beim Anziehen der Befestigungsmutter an einem Lagerteil einer der beiden Knochenverankerungsvorrichtungen mit einem Drehmomentschlüssel;
- Figur 14:: eine perspektivische Gesamtansicht ähnlich Figur 11 des Wirbelsäulenfixationssystems, wobei die Verbindungsplatte mittels zweier Befestigungsmuttern angezogen ist, beim endgültigen Anziehen des Verankerungsteils einer der beiden Knochenverankerungsvorrichtungen;
- Figur 15:: eine perspektivische Ansicht analog Figur 14 mit einem auf ein Zentrierelement einer Knochenverankerungsvorrichtung aufgesetzten, nicht erfindungsgemäßen, Zielgerät;
- Figur 16:: eine teilweise durchbrochene, vergrößerte Ansicht der Gesamtanordnung aus Figur 15;
- Figur 17:: eine Ansicht analog Figur 16 beim Einschrauben einer zusätzlichen Befestigungsschraube mittels des Zielgeräts;
- Figur 18:: eine Ansicht ähnlich Figur 15 jedoch beim Festziehen des Fixierelements mittels eines nicht erfindungsgemäßen Drehmomentschlüssels;
- Figur 19:: eine vergrößerte, teilweise geschnittene Ansicht der in Figur 18 dargestellten Anordnung beim Festziehen des Fixierelements nach Durchtrennen eines Sollbruchbereichs desselben; und
- Figur 20:: eine perspektivische Gesamtansicht des an zwei Wirbelkörpern fixierten Wirbelsäulenfixationssystems.

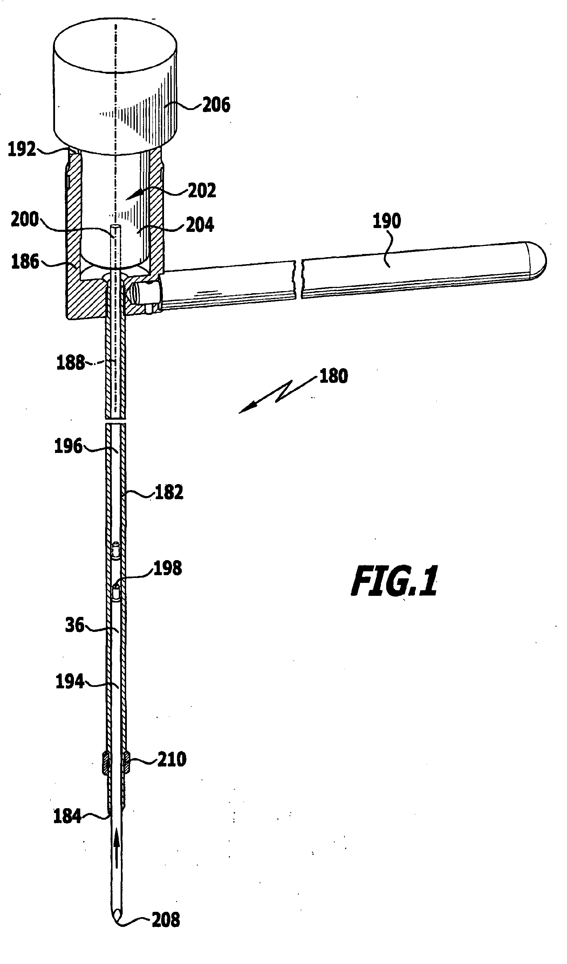

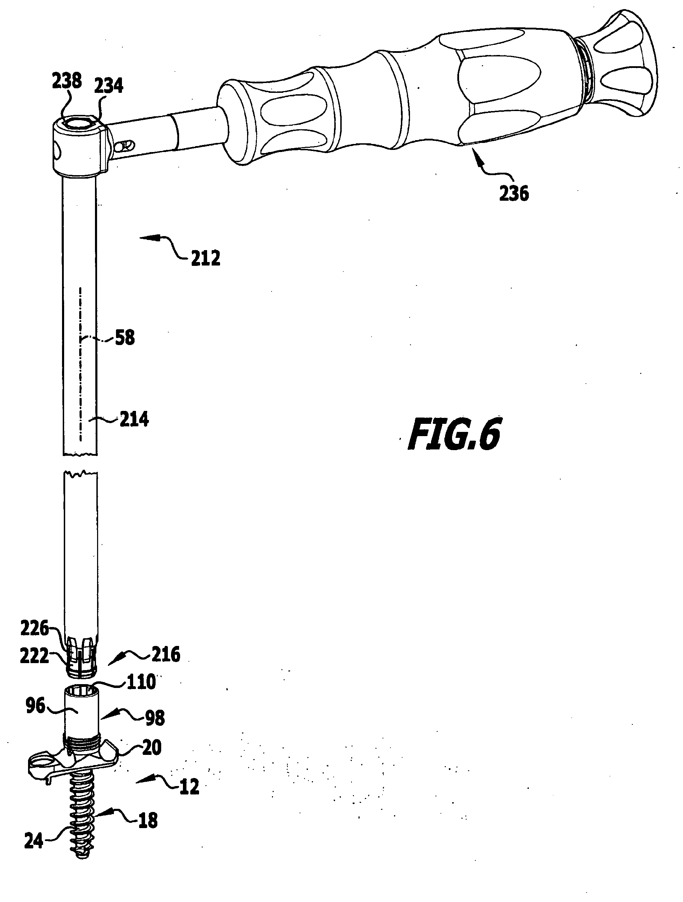

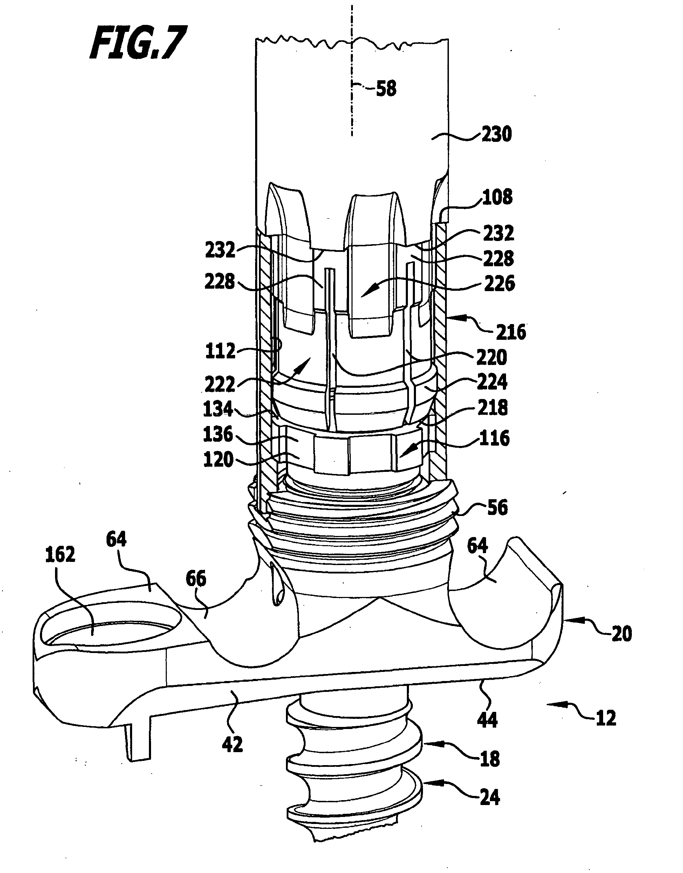

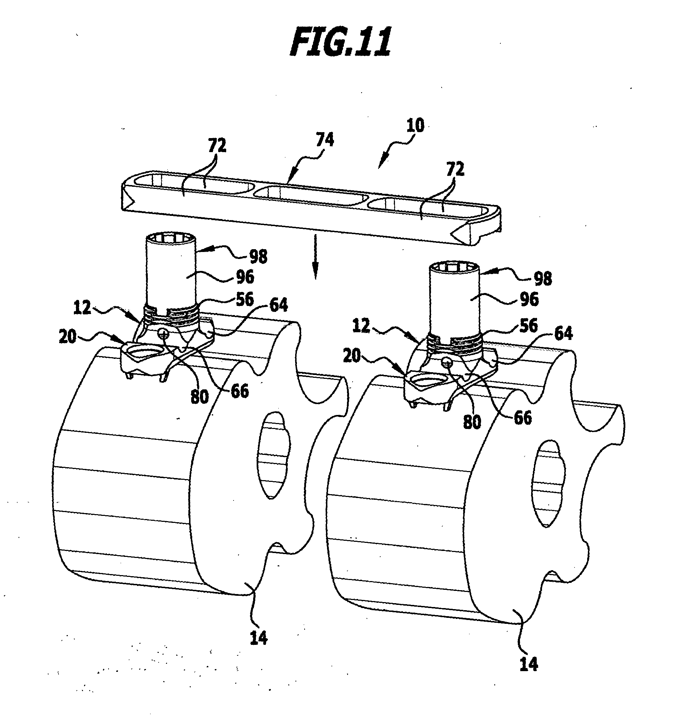

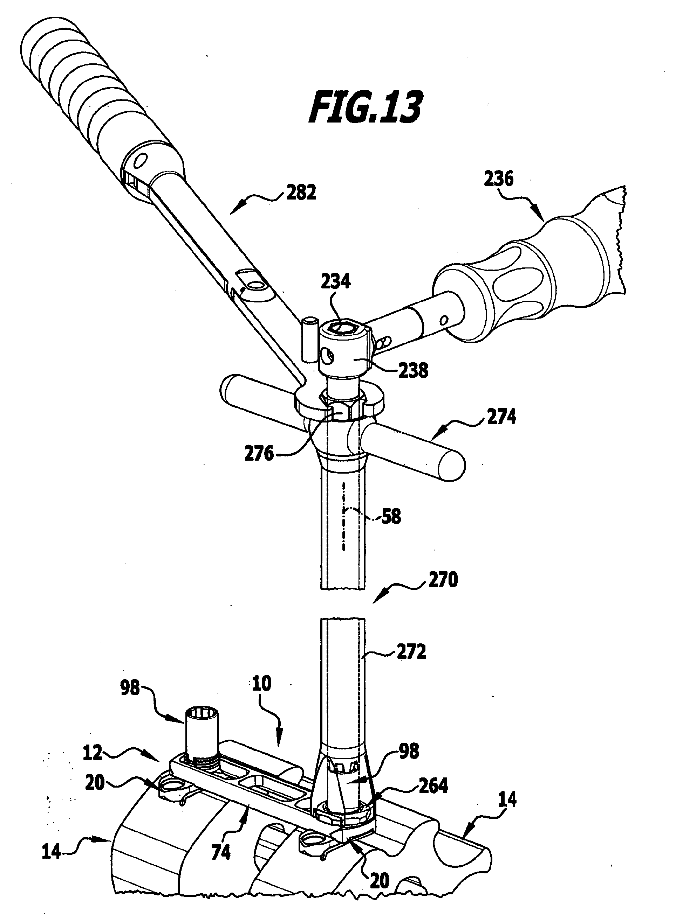

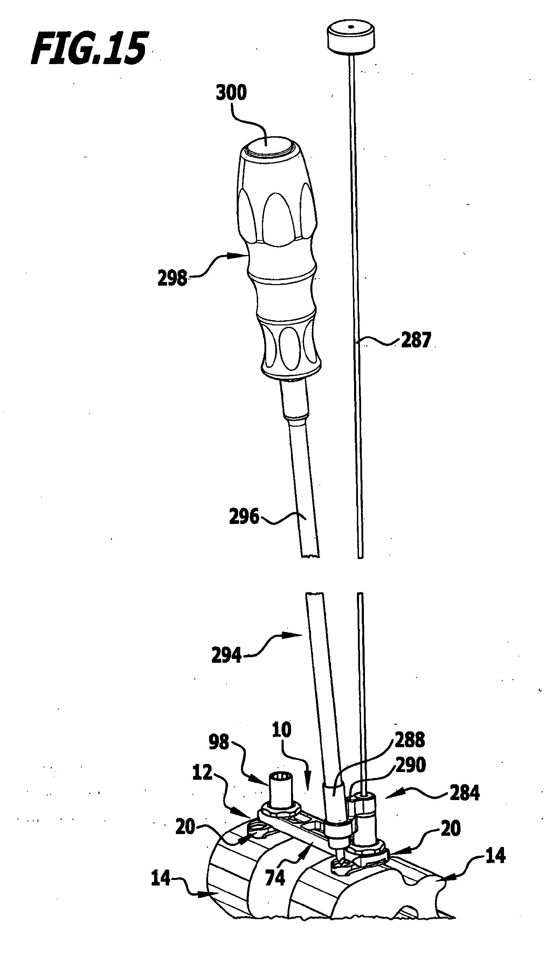

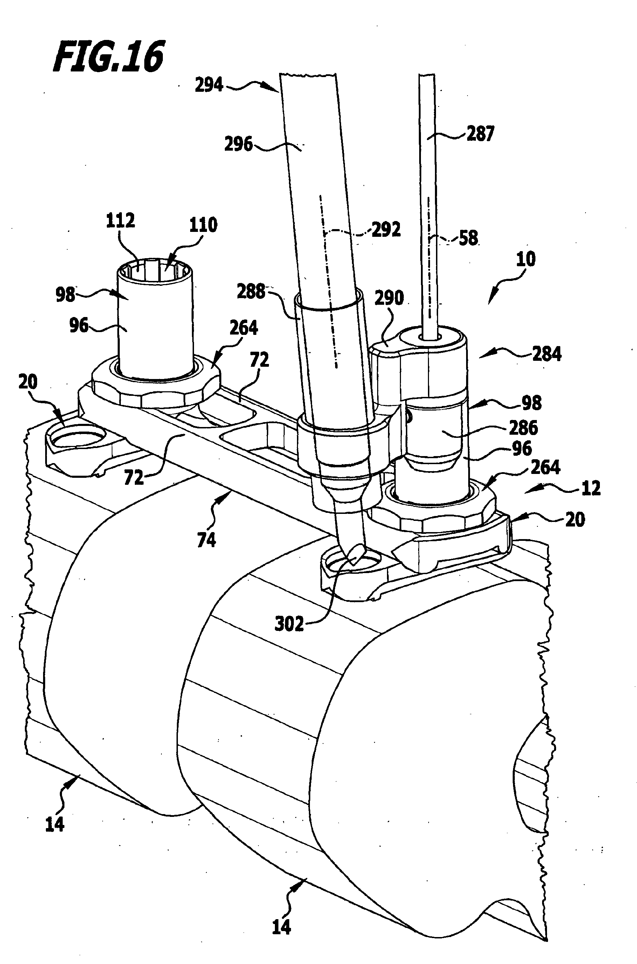

Ein insgesamt mit dem Bezugszeichen 10 versehenes Wirbelsäulenfixationssystem umfassend zwei Knochenverankerungsvorrichtungen 12 ist in den Figuren 1 bis 20 schematisch dargestellt. Es dient zum Festlegen beispielsweise zweier benachbarter Wirbelkörper 14, die in den Figuren schematisch dargestellt sind, relativ zueinander, um zum Beispiel dadurch ein zwischen den Wirbelkörpern 14 definiertes Bandscheibenfach 16 zu entlasten. Die vom Wirbelsäulenfixationssystem 10 umfassten Elemente sowie die zum Implantieren desselben eingesetzten Instrumente werden nachfolgend im Einzelnen mit Bezug zu den Figuren erläutert.

Die Knochenverankerungsvorrichtung 12 umfasst einen Verankerungsteil 18 sowie einen Lagerteil 20. Der Verankerungsteil 18 ist in Form einer eine Längsachse 22 definierenden Knochenschraube 24 ausgebildet, die einen langgestreckten, sich in distaler Richtung konisch etwas verjüngenden Schraubenschaft 26 umfasst, welcher ein Außengewinde 28 trägt, welches in Form eines Knochengewindes ausgebildet ist. Das Außengewinde 28 kann optional selbstschneidend ausgebildet sein. Ein proximales Ende der Knochenschraube 24 wird gebildet durch einen einen Kopfabschnitt definierenden Schraubenkopf 30, welcher einen maximalen Außendurchmesser definiert, der größer ist als ein maximaler Außendurchmesser des Außengewindes 28. Der Schraubenkopf 30 ist zudem mit einem Außengewinde 32 versehen. Optional kann der Schraubenschaft 26 kanuliert sein, das heißt eine sich koaxial zur Längsachse 22 erstreckende Längsbohrung 34 aufweisen, welche, wie nachfolgend näher erläutert wird, der Aufnahme eines K-Drahts 36 dient, um die Knochenschraube 24 in definierter Weise an einem Wirbelkörper 14 festzulegen. Zwischen dem Außengewinde 32 und dem Schraubenkopf 30 erstreckt sich ein kurzer, gewindefreier, zylindrischer Schaftabschnitt 38. Am Schraubenkopf 30 ist eine koaxial zur Längsachse 22 ausgebildete und in proximaler Richtung weisend geöffnete Werkzeugaufnahme 40 ausgebildet. Die Werkzeugaufnahme 40 kann wahlweise in Form eines Innenvielkant oder Innenvielrund, insbesondere in Form einer Torx^{®}-Aufnahme, ausgebildet sein.

Der Lagerteil 20 umfasst einen im Wesentlichen plattenförmigen Grundkörper 42, welcher auf einer Unterseite 44 zwei um wenige Grad relativ zueinander geneigte Flächenbereiche 46 und 47 aufweist. Im Grundkörper 42 ist ein hohlkalottenförmiger Sitz 48 für den Schraubenkopf 30 ausgebildet, dessen Innendurchmesser an den Außendurchmesser des Schraubenkopfs 30 angepasst ist. Der Sitz 48 ist zur Unterseite 44 hin durch eine Durchgangsbohrung 50 eröffnet. Ein Innendurchmesser der Durchgangsbohrung 50 ist wahlweise etwas größer als ein maximaler Außendurchmesser des Außengewindes 28 oder zumindest so bemessen, dass der Schraubenschaft 26, wenn dessen Außengewinde 28 im Außendurchmesser etwas größer ist als die Durchgangsbohrung 50, durch diese hindurchgeschraubt werden kann.

Proximalseitig schließt sich an den Sitz 48 in proximaler Richtung abstehend ein kurzer Hülsenabschnitt 52 an, welcher sowohl mit einem Innengewinde 54 als auch mit einem Außengewinde 56 versehen ist. Das Innengewinde 54 entspricht dem Außengewinde 32 des Schraubenkopfs 30, so dass dieser, der einen größeren Außendurchmesser aufweist als ein freier Innendurchmesser des Hülsenabschnitts 52 im Bereich des Innengewindes 54, durch dieses koaxial zu einer vom Hülsenabschnitt 52 definierten Längsachse 58 eingeschraubt werden kann. Die Längsachse 58 verläuft senkrecht zum Flächenbereich 47. Nach Einschrauben der Knochenschraube 24 mit deren Schraubenkopf 30 durch den Hülsenabschnitt 52 hindurch, ist der Schraubenkopf 30 im Sitz 48 beweglich gehalten. Es wird so ein Kugelgelenk 60 ausgebildet zwischen der Knochenschraube 24 und dem Lagerteil 20. Eine solche Anordnung ist in der Literatur auch bekannt unter der Bezeichnung "Polyaxialschraube", da der Lagerteil 20 aufgrund des Kugelgelenks 60 relativ zum Verankerungsteil 18 um einen Mittelpunkt des Schraubenkopfs 30 polyaxial verschwenkt werden kann.

In den Grundkörper 42 sind ausgehend von dessen Oberseite 62 zwei Verbindungselementaufnahmen 64 und 66 eingearbeitet, welche zumindest abschnittsweise hohlzylindrische Seitenflächen 68 und 70 definieren zur Aufnahme im Querschnitt im Wesentlichen kreisförmiger, stabförmiger Abschnitte 72 eines plattenförmigen Verbindungselements 74 des Wirbelsäulenfixationssystem 10. Von den Verbindungselementaufnahmen 64 und 66 definierte Längsachsen 76 und 78 verlaufen parallel zueinander und liegen jeweils in einer Ebene, die die Längsachse 58 senkrecht schneidet. Im Bereich der Verbindungselementaufnahmen 64 und 66 ist das Außengewinde 56 jeweils teilweise entfernt. Des Weiteren ist im Bereich der Verbindungselementaufnahmen 64 und 66, wo das Außengewinde 56 teilweise entfernt ist, eine sich quer zur Längsachse 58 erstreckende Querbohrung 80 vorgesehen.

Eine in proximaler Richtung weisende, ringförmige Stirnfläche 82 des Hülsenabschnitts 52 ist durch zwei einander diametral gegenüberliegende Aussparungen 84 unterbrochen, welche erste Positionierglieder 86 einer insgesamt mit dem Bezugszeichen 88 versehenen Positioniereinrichtung bilden. Die Aussparungen 84 bilden auf diese Weise Positionieraufnahmen zum formschlüssigen Aufnehmen zweiter Positionierglieder 90, die in Form von korrespondierenden Positioniervorsprüngen 92 ausgebildet sind, die von einer in distaler Richtung weisenden, ringförmigen Stirnfläche 94 eines in Form einer Zentrierhülse 96 ausgebildeten Zentrierelements 98 abstehen. Die Zentrierhülse 96 weist einen Außendurchmesser auf, welcher einem minimalen Außendurchmesser des Hülsenabschnitts 52 im Bereich des Außengewindes 56 entspricht, so dass ein Gewindegang des Außengewindes 56 über eine von der Zentrierhülse 96 definierte zylindrische Außenfläche radial vorsteht.

Das Zentrierelement 98 weist ausgehend von seinem durch die Stirnfläche 94 definierten distalen Ende einen kurzen Hülsenabschnitt 100 auf, welcher einen minimalen Innendurchmesser definiert. Die Zentrierhülse 96 erweitert sich proximalseitig an den Hülsenabschnitt 100 anschließend einstufig, so dass eine in proximaler Richtung weisende, ringförmige Anschlagfläche 102 definiert wird. Ausgehend von der Anschlagfläche 102 erstreckt sich somit ein mittlerer Hülsenabschnitt 104 in proximaler Richtung, der einen maximalen Innendurchmesser der Zentrierhülse 96 definiert. An den mittleren Hülsenabschnitt 104 schließt sich proximalseitig bis zu einem proximalen Ende 108 des Zentrierelements 98 ein proximaler Hülsenabschnitt 106 an. Dieser zeichnet sich dadurch aus, dass er mit einer Innenverzahnung 110 versehen ist, die gebildet wird durch mehrere, sich parallel zur Längsachse 58 erstreckende, streifenförmige Vorsprünge 112, die von einer inneren Wandfläche 114 etwa so weit vorstehen, wie die Anschlagfläche 102 breit ist.

Um das Zentrierelement 98 in definierter Weise mittels der Positioniereinrichtung 88 am Lagerteil 20 festzulegen, ist ein insgesamt mit dem Bezugszeichen 116 versehenes Fixierelement vorgesehen. Es ist insgesamt einstückig ausgebildet und definiert einen distalen Fixierelementabschnitt 118 sowie einen proximalen Fixierelementabschnitt 120, die durch einen Sollbruchbereich 122 voneinander getrennt sind.

Der distale Fixierelementabschnitt 118 definiert eine in distaler Richtung weisende, ringförmige, hohlkalottenförmige Fixierfläche 124, deren Krümmung an die kugelförmige Außenkontur des Schraubenkopfs 30 angepasst ist. Proximalseitig der Fixierfläche 124 schließt sich ein ringförmiger, in Richtung auf die Längsachse 58 hin weisender Ringvorsprung 126 an, welcher einen minimalen Innendurchmesser des Fixierelements 116 definiert. Proximalseitig des Ringvorsprungs 126 erweitert sich das Fixierelement 116 im Inneren im Wesentlichen einstufig. Bis zum Sollbruchbereich 122 ist der distale Fixierelementabschnitt 118 im Inneren mit einer eine Werkzeugelementaufnahme 128 definierenden Verzahnung 130 versehen, die in Form eines Innenvielrunds ausgebildet ist, optional auch in Form eines Innenvielkant ausgebildet sein kann. Auf seiner Außenseite ist das Fixierelement 116 mit einem Außengewindeabschnitt 132 versehen, welcher korrespondierend zum Innengewinde 54 des Hülsenabschnitts 52 ausgebildet ist. Dies ermöglicht es, den distalen Fixierelementabschnitt 118 mit dem Innengewinde 54 des Hülsenabschnitts 52 zu verschrauben.

Ausgehend vom Sollbruchbereich 122 erweitert sich ein Innendurchmesser des Fixierelements 116 derart, dass es vollständig verzahnungsfrei ist. Der proximale Fixierelementabschnitt 120 ist somit nicht geeignet, um mit einem Einschraubwerkzeug in Eingriff gebracht zu werden und so ein Drehmoment auf das Fixierelement 116 zu übertragen. Ausgehend von einer proximalen Endfläche 134 ist ein in radialer Richtung von der Längsachse 58 weg weisender Ringflansch 136 ausgebildet, welcher mit mehreren Nuten 138 versehen ist zur Ausbildung einer Außenverzahnung 140, die zur Innenverzahnung 110 korrespondierend ausgebildet ist. Ein maximaler Außendurchmesser der Außenverzahnung 140 ist wenig kleiner als ein Innendurchmesser des mittleren Hülsenabschnitts 104 und entspricht einem maximalen Innendurchmesser des proximalen Hülsenabschnitts 106 im Bereich eines Zahngrunds 142 der Innenverzahnung 110.

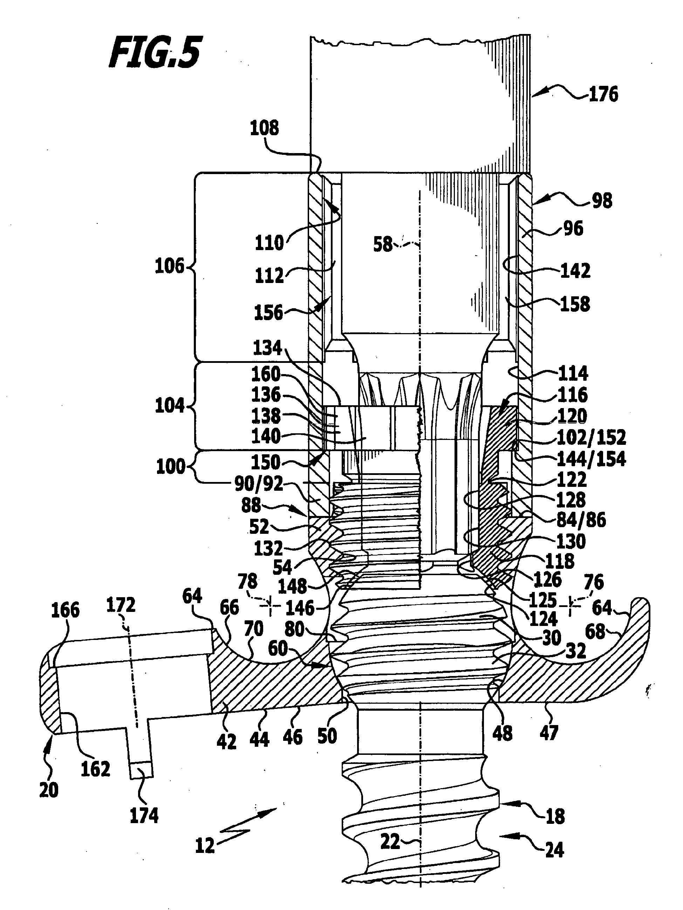

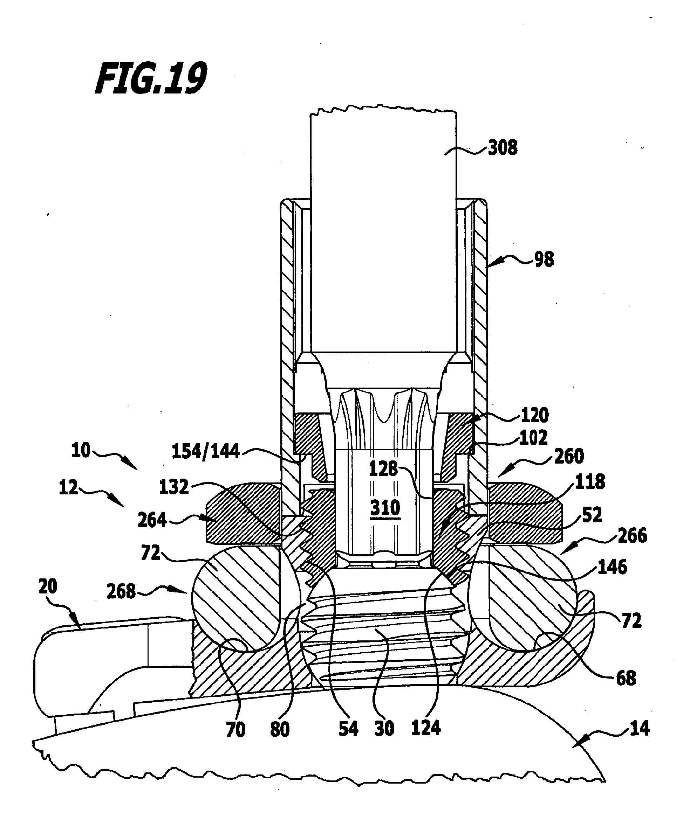

Der Ringflansch 136 definiert eine in distaler Richtung weisende, ringförmige Anschlagfläche 144, welche in einer Montagestellung der Knochenverankerungsvorrichtung 12, wie sie schematisch in Figur 5 dargestellt ist, an der Anschlagfläche 102 anliegt. Das Fixierelement 116 ist insgesamt so dimensioniert, dass in der Montagestellung die Fixierfläche 124 von einem Fixierflächenbereich 146 des Schraubenkopfs 30 beabstandet ist. Der Fixierflächenbereich 146 ist dabei definiert als der Flächenbereich einer Oberfläche des Schraubenkopfs 30, an welchem die Fixierfläche 124 anliegt, wenn der Lagerteil 20 und der Verankerungsteil 18 gegeneinander gespannt sind und eine Implantationsstellung definieren, wie dies schematisch in Figur 19 dargestellt ist. In der Montagestellung ist die Fixierfläche 124 vom Fixierflächenbereich 146 durch einen Spalt 148 beabstandet.

Die Montagestellung wird definiert durch eine insgesamt mit dem Bezugszeichen 150 versehene Kopplungseinrichtung, welche ein erstes Kopplungsglied 152 in Form der Anschlagfläche 102 sowie ein zweites Kopplungsglied 154 in Form der Anschlagfläche 144 umfasst. Die ersten und zweiten Kopplungsglieder 152 und 154 liegen in einer Kopplungsstellung aneinander an und stehen auf diese Weise in Eingriff miteinander. Sie stellen sicher, dass die Fixierfläche 124 vom Fixierflächenbereich 146 beabstandet ist solange der Sollbruchbereich 122 unbeschädigt oder unzerstört ist. Die Kopplungseinrichtung 150 bildet somit gleichzeitig eine Bewegungsbegrenzungseinrichtung zumindest für den proximalen Fixierelementabschnitt 120 in distaler Richtung. In proximaler Richtung wird eine Bewegung des Fixierelements 116 begrenzt durch die Endfläche 134, die an in distaler Richtung weisenden Seitenflächen der Vorsprünge 112 anschlägt, wenn das Fixierelement 116 um die Längsachse 58 so gedreht ist, dass die Innenverzahnung 110 und die Außenverzahnung 140 nicht in Eingriff gebracht werden können. Auf diese Weise ist das Fixierelement 116 mit seinem proximalen Fixierelementabschnitt 120 unverlierbar am Zentrierelement 98 gesichert. Mit anderen Worten bilden die Innenverzahnung 110 und die Außenverzahnung 140 erste und zweite Sicherungsglieder 158 und 160 einer insgesamt mit dem Bezugszeichen 156 versehenen Sicherungseinrichtung zum Sichern des proximalen Fixierelementabschnitts 120 am Zentrierelement 98.

Das Zentrierelement 98 kann in der beschrieben Weise mit dem Fixierelement 116 und durch in Eingriff Bringen der ersten und zweiten Positionierglieder 86 und 90 derart gegen den Lagerteil 20 gespannt werden, dass die Stirnflächen 94 und 82 aneinander anliegen. Eine Verdrehung des Zentrierelements 98 um die Längsachse 58 relativ zum Lagerteil 20 ist dann unmöglich.

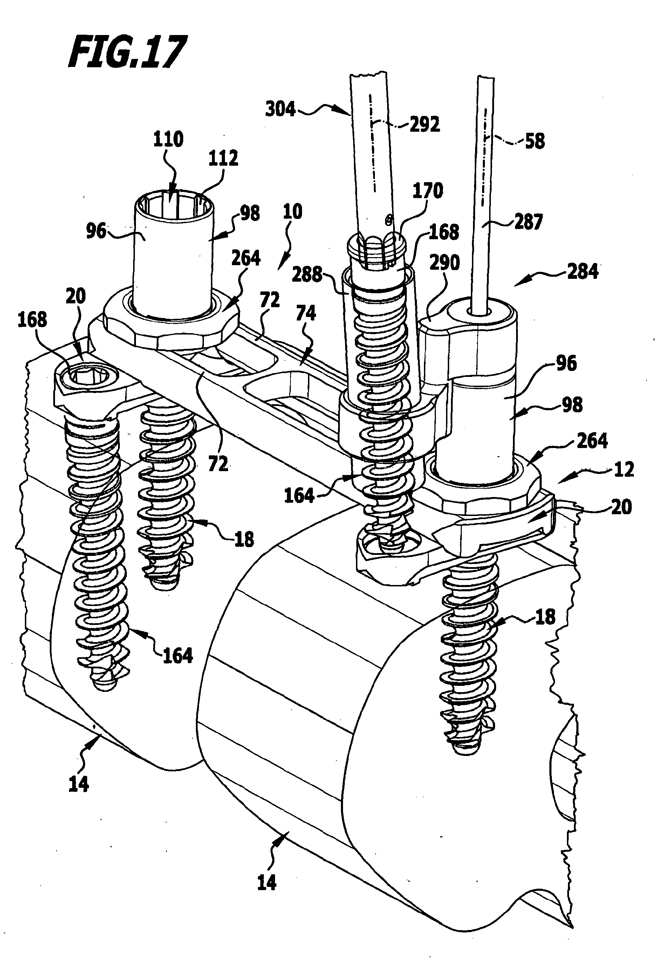

Der Grundkörper 42 des Lagerteils 20 kann optional unsymmetrisch ausgebildet sein und benachbart der Verbindungselementaufnahme 66 zusätzlich eine Befestigungselementaufnahme 162 in Form einer Bohrung mit sich in distaler Richtung einstufig verjüngendem Innendurchmesser umfassen, welche Bohrung zur Aufnahme eines zusätzlichen Befestigungselements 164 dient, um das Lagerteil 20 verdrehsicher am Wirbelkörper 14 festzulegen. Durch die einstufige Verjüngung des Innendurchmessers der Befestigungselementaufnahme 162 wird eine in proximaler Richtung weisende, ringförmige Anschlagfläche 166 definiert, welche einen Anschlag für einen sich radial vom Kopf 168 des Befestigungselements 164 vorspringenden Ringflansch 170 bildet. Das Befestigungselement 164 kann insbesondere in Form einer Knochenschraube ausgebildet sein, wie dies schematisch in Figur 17 dargestellt ist. Eine Längsachse 172 der Befestigungselementaufnahme 162 verläuft senkrecht zum Flächenbereich 46 und ist somit um wenige Grad gegenüber der Längsachse 58 geneigt.

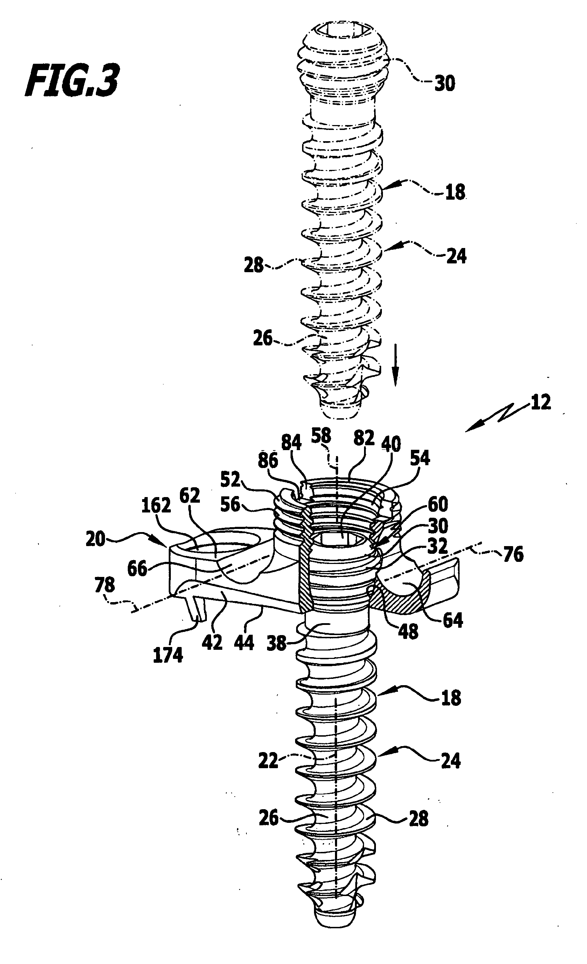

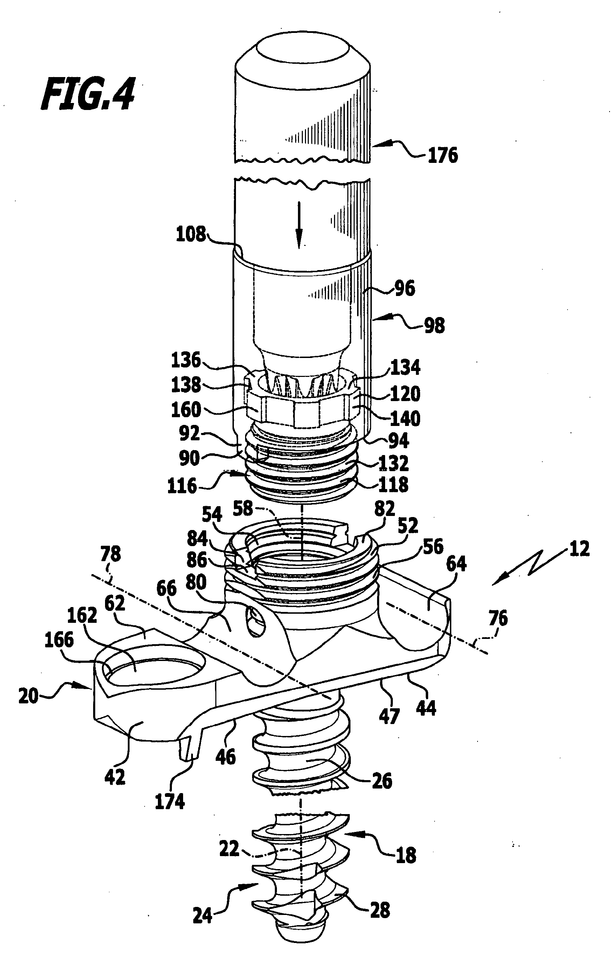

Optional kann von der Unterseite 44 des Grundkörpers 42 ein Verankerungselement 174 abstehen, beispielsweise in Form eines Haltedorns, wie dieser schematisch zum Beispiel in den Figuren 3 und 4 dargestellt ist, welcher eine zusätzliche Verdrehsicherung bildet, wenn er in einen Wirbelkörper 14 eindringt.

Die Knochenverankerungsvorrichtung 12 wird als Einheit vormontiert und umfasst den Verankerungsteil 18 und den Lagerteil 20 sowie optional das Zentrierelement 98 und das Fixierelement 116. Wie in Figur 3 dargestellt, wird zunächst der Schraubenschaft 26 mit seinem distalen Ende von proximal her durch den Hülsenabschnitt 52 hindurch geführt, bis der Schraubenkopf 30 am Sitz 48 anliegt.

Des Weiteren wird das Fixierelement 116 mit seinem distalen Fixierelementabschnitt 118 von proximal her kommend in das Ende 108 des Zentrierelements 98 eingeführt. Das Fixierelement 116 wird bezogen auf die Längsachse 58 relativ zum Zentrierelement 98 so ausgerichtet, dass die Innenverzahnung 110 und die Außenverzahnung 140 ineinander greifen können und das Fixierelement 116 in distaler Richtung so weit vorgeschoben werden kann, bis die ersten und zweiten Kopplungsglieder 152 und 154 aneinander anliegen. Mittels eines Einschraubwerkzeugs 176, welches ein distales Werkzeugende 178 aufweist, welches korrespondierend zur Werkzeugelementaufnahme 128 ausgebildet ist, kann der Außengewindeabschnitt 132 mit dem Innengewinde 54 verschraubt werden, und zwar genau so weit, bis die Stirnflächen 82 und 94 aneinander anliegen. Dabei ist darauf zu achten, dass der Sollbruchbereich 122 nicht zerstört wird. Das Einschraubwerkzeug 176 ist daher vorzugsweise mit einer Drehmomentbegrenzung ausgestattet, so dass lediglich ein Drehmoment aufgebracht wird, welches kleiner ist als ein Bruchmoment des Sollbruchbereichs 122.

Nach Entfernen des Einschraubwerkzeugs 176 ist die Knochenverankerungsvorrichtung 12 mit Zentrierelement 98 vormontiert, um in einen Wirbelkörper 14 implantiert zu werden.

Möglichkeiten der Implantation des Wirbelsäulenfixationssystems 10 mittels geeigneter und dafür vorgesehener Instrumente werden nachfolgend Schritt für Schritt anhand der Figuren 1 bis 20 im Einzelnen erläutert.

Falls als Verankerungsteil 18 eine kanulierte Knochenschraube 24 verwendet wird, wie sie bereits oben beschrieben wurde, kann in einem optionalen ersten Schritt nach Eröffnen eines Zugangs zum menschlichen Körper ein insgesamt mit dem Bezugszeichen 180 bezeichnetes K-Draht-Einsetzinstrument zum Einsatz kommen, um den K-Draht 36 im Wirbel 14 zu platzieren. Das K-Draht-Einsetzinstrument 180 umfasst eine langgestreckte Führungshülse 182 mit einem schneidenden distalen Ende 184. Ein proximales Ende des K-Drahteinsetzinstruments 180 bildet ein koaxial zu einer Längsachse 188 der Führungshülse 182 drehfest montierter Hohlzylinder 186, von dem in einer Richtung quer zur Längsachse 188 ein Haltegriff 190 absteht. Der Hohlzylinder 186 ist in proximaler Richtung weisend geöffnet und definiert eine in proximaler Richtung weisende Ringfläche 192, die als Schlagfläche dient, um das distale Ende 184 in den Wirbelkörper 14 zu treiben.

Der K-Draht 36 ist zweiteilig ausgebildet und umfasst einen distalen Abschnitt 194 und einen proximalen Abschnitt 196, die über eine Kupplungseinrichtung 198 miteinander verbunden sind. Insbesondere kann die Kupplungseinrichtung 198 in Form einer Schraubverbindung ausgebildet sein, um den distalen Abschnitt 194 und den proximalen Abschnitt 196 miteinander zu verschrauben. An einem proximalen Ende 200 des proximalen Abschnitts 196 ist ein Kolbenkörper 202 angeordnet, welcher einen distalen Kolbenabschnitt 204 und einen proximalen Kolbenabschnitt 206 umfasst. Der distale Kolbenabschnitt 204 ist derart dimensioniert, dass er formschlüssig in den Hohlzylinder 186 von proximal her kommend eingeführt werden kann, und zwar derart, dass der K-Draht 36 durch die Führungshülse 182 mit seinem distalen, angespitzten Ende 208 hindurch geführt und über das Ende 184 vorgeschoben werden kann. Der proximale Kolbenabschnitt 206 ist im Außendurchmesser größer als der Hohlzylinder 186 und bildet einen Anschlag für den Hohlzylinder 186, wodurch eine Bewegung des K-Drahts 36 in distaler Richtung begrenzt werden kann. Das Ende 208 steht maximal weit über das Ende 184 vor, wenn der proximale Kolbenabschnitt 206 an der Ringfläche 192 anliegt.

Um eine Einschlagtiefe der Führungshülse 182 in den Wirbelkörper zu begrenzen, ist etwas beabstandet vom Ende 184 ein in radialer Richtung von der Längsachse 188 weg weisender und vorspringender Ringflansch 210 angeordnet.

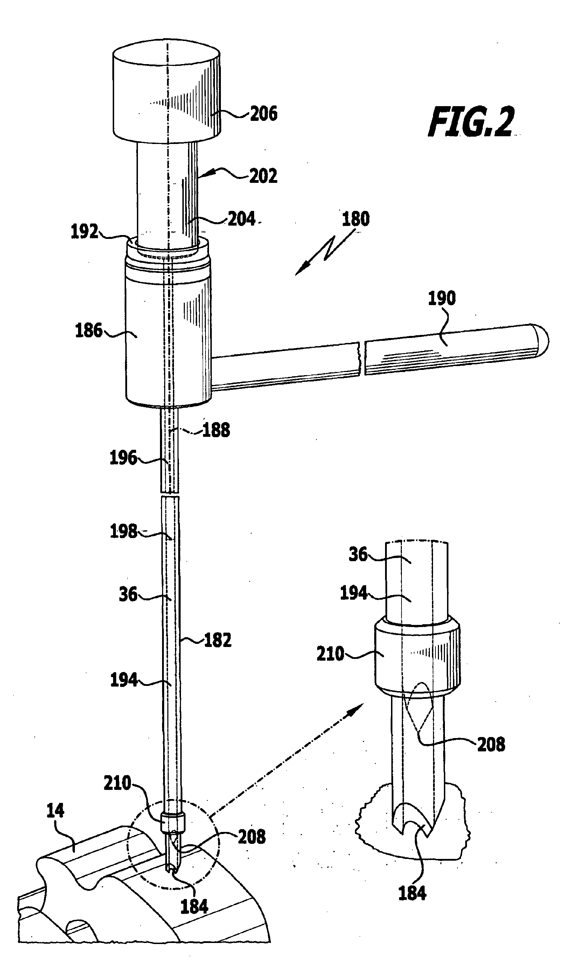

Wie in Figur 2 schematisch dargestellt, wird zunächst das K-Draht-Einsetzinstrument 180 vorzugsweise ohne den K-Draht 36 in den Wirbelkörper 14 eingeschlagen und danach in der beschriebenen Weise der K-Draht 36 durch die Führungshülse 182 hindurch eingeführt und in den Wirbelkörper 14 eingetrieben. Ist der K-Draht 36 in gewünschter Weise im Wirbelkörper 14 platziert, wird der proximale Abschnitt 196 vom distalen Abschnitt 194 getrennt, beispielsweise abgeschraubt. Anschließend wird auch das K-Draht-Einsetzinstrument 180 aus dem Wirbelkörper 14 entfernt. Im Wirbelkörper 14 verbleibt dann lediglich der distale Abschnitt 194 des K-Drahts 36.

Als nächstes wird die wie oben beschrieben vormontierte Knochenverankerungsvorrichtung 12 mit Zentrierhülse 96 für die Implantation vorbereitet. Um sie in den Körper des Patienten einführen.zu können, wird sie zunächst mit einem insgesamt mit dem Bezugszeichen 212 versehenen Halteinstrument verbunden. Das Halteinstrument 212 umfasst eine koaxial zur Längsachse 58 mit der Zentrierhülse 96 verbindbare Führungshülse 214, deren Außendurchmesser dem Außendurchmesser des Zentrierelements 98 entspricht. Mittels einer insgesamt mit dem Bezugszeichen 216 bezeichneten Verbindungseinrichtung lässt sich die Führungshülse 214 mit dem Zentrierelement 98 axial und drehfest verbinden. Mehrere über den Umfang der Führungshülse 214 verteilte, ausgehend von einem distalen Ende 218 derselben parallel zur Längsachse 58 eingebrachte Schlitze 220 definieren zwischen sich Federarme 222. Durch die Schlitze 220 wird ein benachbart des Endes 218 in radialer Richtung von der Längsachse 58 weg weisend vorspringender Ringflansch 224 unterbrochen. Die Federarme 222 mit dem abschnittsweise angeformten Ringflansch 224 bilden Rastelemente der in Form einer Rastverbindung ausgebildeten Verbindungseinrichtung 216. Etwas beabstandet vom Ringflansch 224 ist proximalseitig desselben an der Führungshülse 214 eine Außenverzahnung 226 ausgebildet, und zwar mit mehreren über den Umfang verteilten, sich parallel zur Längsachse 58 erstreckenden Vorsprüngen 228. Die Außenverzahnung 226 ist korrespondierend zur Innenverzahnung 110 ausgebildet. Die Vorsprünge 228 sind bezogen auf eine äußere Oberfläche 230 der Führungshülse 214 etwas zurückversetzt, so dass jedem Vorsprung 228 eine in distaler Richtung weisende, sich proximalseitig daran anschließende Anschlagfläche 232 zugeordnet ist.

Die Führungshülse 214 wird wie folgt mit dem Zentrierelement 98 verbunden. Das Ende 218 wird von proximal her kommend in die Zentrierhülse 96, die bereits mit dem Fixierelement 116 am Lagerteil 20 festgelegt ist, eingeführt. Dabei gleiten die verbliebenen Abschnitte des Ringflansches 224 auf den Federarmen 222 an der Innenverzahnung 110 auf und werden etwas in Richtung auf die Längsachse 58 hin vorgespannt. Die Führungshülse 214 wird dabei so um die Längsachse 58 gedreht, dass die Außenverzahnung 226 mit der Innenverzahnung 110 in Eingriff gebracht werden kann. So ist es möglich, die Führungshülse 214 so weit in distaler Richtung vorzuschieben, bis die Anschlagflächen 232 am proximalen Ende 108 der Zentrierhülse 96 anschlagen. Die Federarme 222 sind so lang und der Ringflansch 224 entsprechend positioniert, dass dann, wenn die Anschlagflächen 232 am Ende 108 in Anlage kommen, die Federarme 222 distalseitig der Innenverzahnung wieder radial von der Längsachse 58 weg ausfedern können. Die Führungshülse 214 und die Zentrierhülse 96 sind auf diese Weise rastend axial und drehfest miteinander verbunden.

Ein proximales Ende der Führungshülse 214 ist mit einem Außensechskant 234 versehen, welcher ein Kopplungsglied bildet, um die Führungshülse 214 mit einem Handgriff 236 zu verbinden. Der Handgriff 236 steht vorzugsweise in einer Richtung senkrecht zur Längsachse 58 von der Führungshülse 214 ab. Er ist mit einem entsprechenden Kupplungskörper 238 ausgestattet, welcher eine kraft- und formschlüssige Verbindung mit dem Außensechskant 234 der Führungshülse 214 ermöglicht. Wahlweise kann der Handgriff 236 lösbar verbindbar oder dauerhaft verbunden mit der Führungshülse 214 vorgesehen sein.

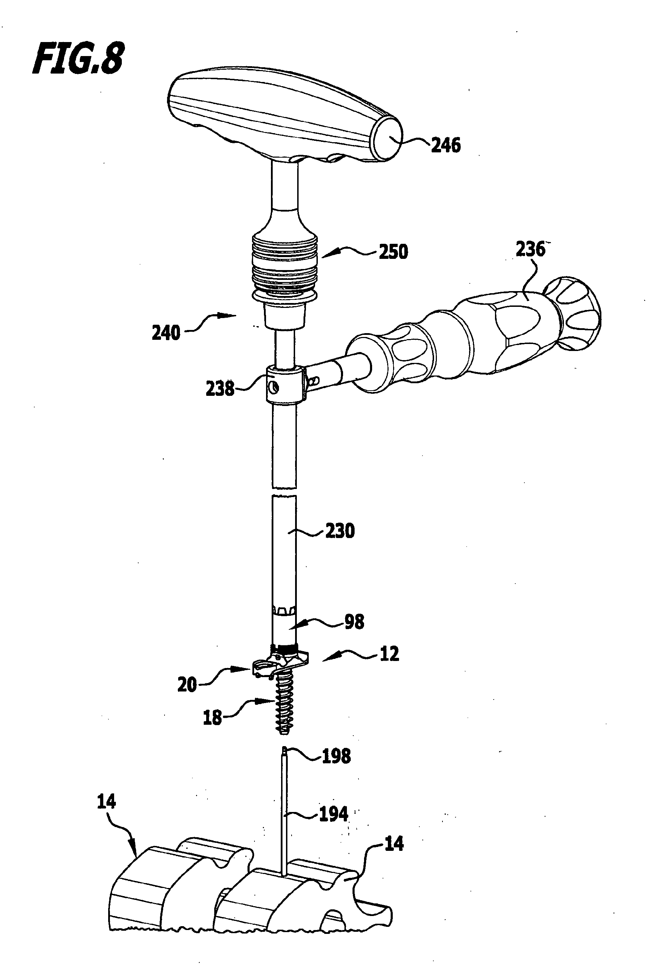

Nach dem Verbinden des Halteinstruments 212 mit der Knochenverankerungsvorrichtung 12 kann ein Operateur die gesamte Einheit bequem am Handgriff 236 halten, die Knochenschraube 24 in den Patientenkörper einführen und auf den K-Draht 36 auffädeln, wie dies schematisch in Figur 8 dargestellt ist. Die Knochenverankerungsvorrichtung 12 nimmt immer noch die Montagestellung ein, das heißt der Verankerungsteil 18 und der Lagerteil 20 sind kugelgelenkig miteinander verbunden.

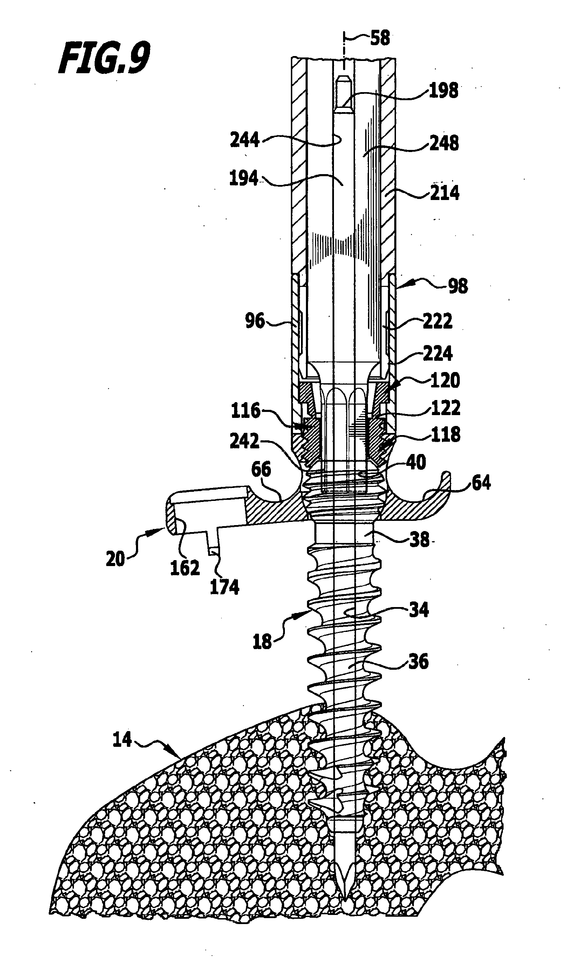

Um die Knochenverankerungsvorrichtung 12 noch besser führen zu können, kann der Operateur bereits jetzt optional ein Einschraubwerkzeug 240 zum Einschrauben der Knochenschraube 24 in den Wirbelkörper von proximal her kommend in die Führungshülse 214 einführen. Das Einschraubwerkzeug 240 weist ein distales Werkzeugende 242 auf, welches korrespondierend zur Werkzeugaufnahme 40 ausgebildet und in diese von proximal her kommend einführbar ist. Dies ist schematisch in Figur 9 dargestellt.

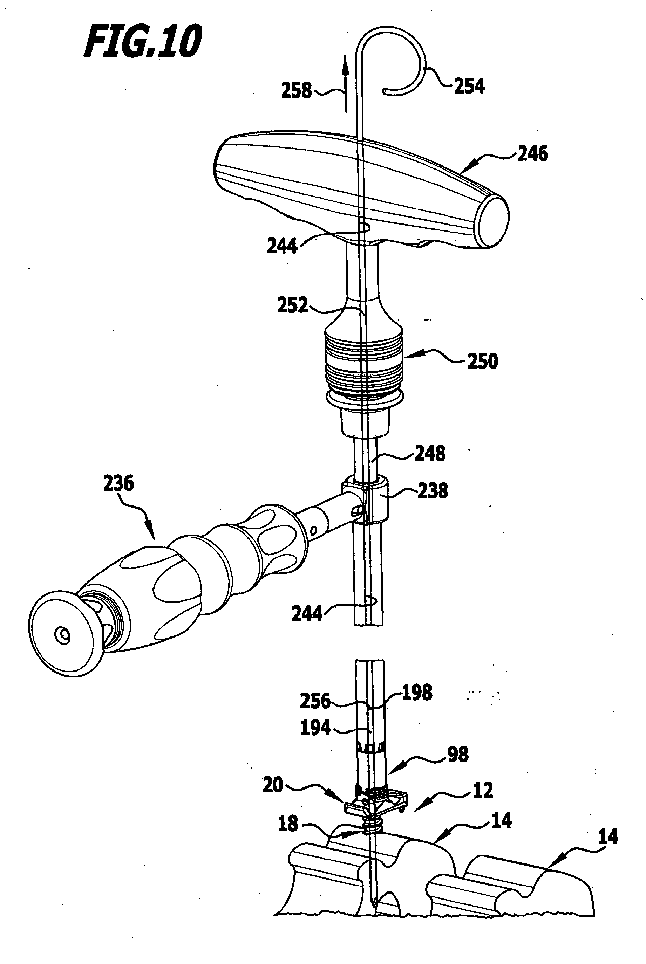

Wie in den Figuren 9 und 10 schematisch dargestellt, ist das Einschraubwerkzeug 240 über seine gesamte Länge mit einem Drahtkanal 244 versehen, welcher koaxial zur Längsachse 58 verläuft. Das Werkzeugende 242 bildet ein distales Ende eines Schafts 248 des Einschraubwerkzeugs 240, an dessen proximalem Ende eine insgesamt mit dem Bezugszeichen 250 versehene Drehmomentbegrenzungseinheit angeordnet ist. Proximalseitig derselben ist ein T-förmiger Griff 246 ausgebildet.

Ein Operateur kann nun, wie in den Figuren 8 und 9 schematisch dargestellt, mit einer Hand den Handgriff 236 halten und mittels des Einschraubwerkzeugs 240 durch Halten des Griffs 246 die Knochenverankerungsvorrichtung 12 an den K-Draht 36 heran und über diesen führen, bis ein distales Ende der Knochenschraube 24 mit dem Wirbelkörper 14 in Kontakt kommt. Zum Eintreiben der Knochenschraube 24 in den Wirbelkörper 14 dreht der Operateur den Griffs 246 im Uhrzeigersinn bis die Knochenschraube 24 sicher im Wirbelkörper 14 platziert ist.

Sobald die Knochenschraube 24 selbst im Wirbelkörper 14 hält, kann der K-Draht 36, das heißt dessen distaler Abschnitt 194 wieder entfernt werden. Zu diesem Zweck wird von proximal her kommend ein Abziehdraht 252 mit umgebogenem proximalen Ende 254 durch den Drahtkanal 244 eingeführt. Ein distales Ende 256 des Abziehdrahts 252 ist ausgebildet, um mit dem proximalen Ende des distalen Abschnitts 194 verbunden zu werden, beispielsweise durch Verschrauben. Nach Verbinden des distalen Abschnitts 194 mit dem Abziehdraht 252 kann diese neu entstandene Drahteinheit vom Operateur durch Ziehen am Ende 254 in Richtung des Pfeils 258 in proximaler Richtung aus dem Wirbelkörper 14 herausgezogen werden. Des Weiteren können das Einschraubwerkzeug 240 und das Halteinstrument 212 von der Knochenverankerungsvorrichtung 12 entfernt werden.

In analoger Weise wird eine weitere Knochenverankerungsvorrichtung 12 in den benachbarten Wirbelkörper 14 eingebracht. Über die beiden Zentrierelemente 98 der beiden in den benachbarten Wirbelkörpern 14 verankerten Knochenverankerungsvorrichtungen 12 kann nun das plattenförmige Verbindungselement 74 geschoben werden, und zwar derart, dass in jeder Verbindungselementaufnahme 64 beziehungsweise 66 der beiden Lagerteile 20 jeweils ein Abschnitt 72 des Verbindungselements 74 eingreift.

Um die Wirbelkörper 14 in einem vom Operateur gewünschten Abstand voneinander festlegen zu können, muss das Verbindungselement 74 mit der Knochenverankerungsvorrichtung 12 fest verbunden werden. Hierzu dient eine insgesamt mit dem Bezugszeichen 260 bezeichnete Klemmeinrichtung . Diese umfasst eine mit einem Innengewinde 262 versehene Mutter 264, wobei das Innengewinde 262 korrespondierend zum Außengewinde 56 des Hülsenabschnitts 52 ausgebildet ist. Wird die Mutter 264 auf den Hülsenabschnitt 52 aufgeschraubt, so können die Abschnitte 72 mittels der Mutter 264 in die Verbindungselementaufnahmen 64 und 66 gedrückt und darin klemmend gehalten werden. Dabei verkleinert die Mutter 264 quer zu von den Verbindungselementaufnahmen 64 und 66 definierten Längsachsen 76 und 78 eröffnete Einführöffnungen 266 beziehungsweise 268 in einer Klemmstellung mindestens teilweise. Dies ist schematisch in Figur 19 gut zu erkennen.

Zum Einsetzen der Mutter 264 dient ein insgesamt mit dem Bezugszeichen 270 bezeichnetes Schraubwerkzeug, welches eine sich koaxial zur Längsachse 58 erstreckende Hülse 272 umfasst. Ein Innendurchmesser der Hülse 272 ist so dimensioniert, dass die Führungshülse 214 des Halteinstruments 212 durch die Hülse 272 hindurch geführt werden kann. Ein proximales Ende des Schraubwerkzeugs 270 ist in Form eines T-Griffs 274 ausgebildet und weist einen in proximaler Richtung abstehenden, koaxial zur Längsachse 58 ausgebildeten und fest mit der Hülse 272 verbundenen Außensechskant 276 auf. Ein distales Ende 280 der Hülse 272 ist in Form einer Mutternaufnahme 278 ausgebildet, in welche die Mutter 264 eingesetzt werden kann. Vorzugsweise ist die Mutternaufnahme 278 derart ausgebildet, dass die Mutter 264 mit der Mutternaufnahme 278 verrastbar ist, um zu vermeiden, dass die Mutter 264 beim Einführen in den Körper des Patienten aus der Mutternaufnahme 278 herausfallen kann.

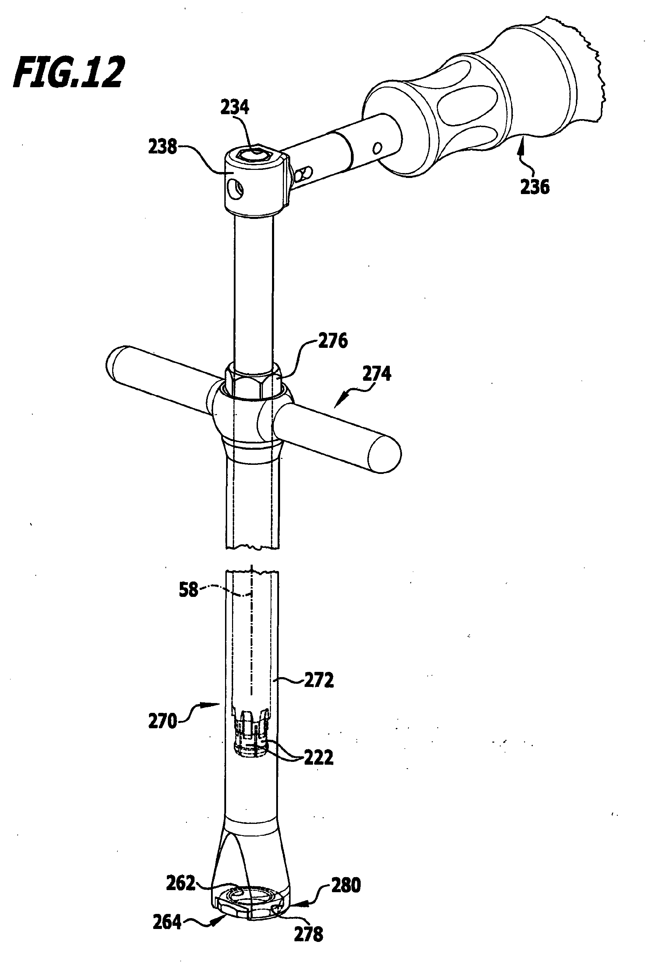

Wie schematisch in Figur 12 dargestellt, kann nun die in die Mutternaufnahme 278 eingesetzte Mutter 264 mit dem Schraubwerkzeug 270 über die Zentrierhülse 96 geführt und durch Drehen des T-Griffs 274 im Uhrzeigersinn mit dem Hülsenabschnitt verschraubt werden. Ist die Mutter 264 mittels des Schraubwerkzeugs 270 an das Verbindungselement 74 herangeführt, wird wiederum das Halteinstrument 212 in der bereits oben beschriebenen Weise mittels der Verbindungseinrichtung 216 mit dem Zentrierelement 98 gekoppelt. Der Handgriff 236 steht nun proximalseitig aus der Hülse 272 heraus. Das Halteinstrument 212 dient dazu, das mittels eines Drehmomentsschlüssels 282 in den Außensechskant 276 eingeleitete Anzugsmoment zum Festziehen der Mutter 264 gegenzuhalten. Sobald der Drehmomentschlüssel 282 bei Überschreiten des voreingestellten Anzugsmoments auslöst, wird dieser entfernt. Danach werden das Halteinstrument 212 und das Schraubwerkzeug 270 von der Knochenverankerungsvorrichtung 12 gelöst und entfernt.

Analog zur beschriebenen Vorgehensweise wird eine weitere Mutter 264 mit der anderen Knochenverankerungsvorrichtung 12 verschraubt. In den Figuren nicht dargestellt ist, dass, falls erforderlich, Distraktionswerkzeuge eingesetzt werden können, um die benachbarten Wirbelkörper 14 in gewünschter Weise zu distrahieren.

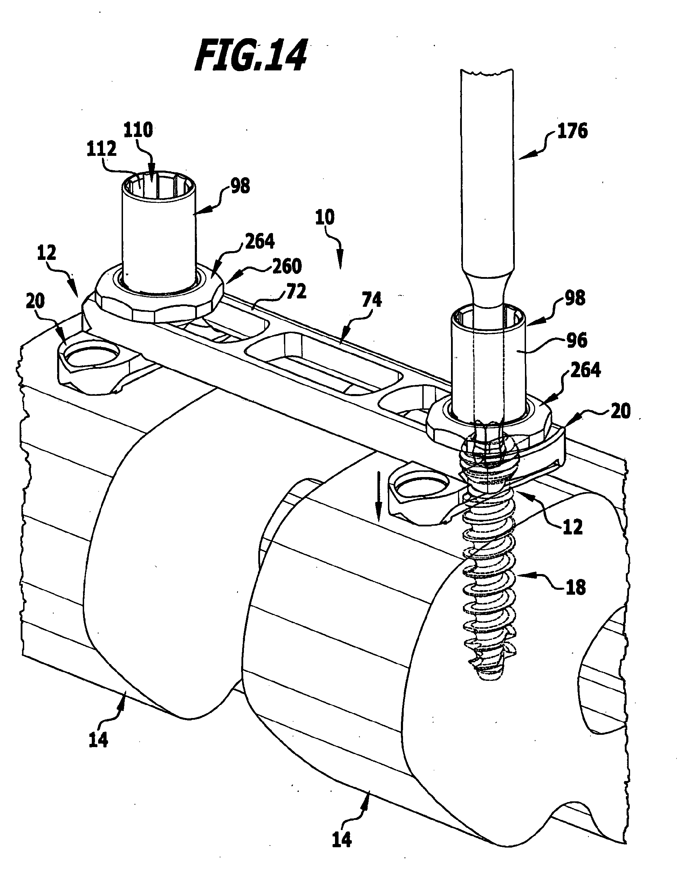

Wie schematisch in Figur 14 dargestellt, sind nun die beiden Knochenverankerungsvorrichtung 12 über das Verbindungselement 74 fest miteinander verbunden. Nach wie vor können jedoch noch die Lagerteile 20 und die Verankerungsteile 18 relativ zueinander verschwenkt werden.

Da die Lagerteile 20 mittels der Knochenschraube 24 vorzugsweise nur so weit in die Wirbelkörper 14 eingeschraubt wurden, dass die Verankerungselemente 174 noch nicht in die Wirbelkörper 14 eingreifen, müssen zum endgültigen Festlegen der Lagerteile an den Wirbelkörpern 14 die Knochenschrauben 24 noch weiter in die Wirbelkörper 14 eingeschraubt werden. Zu diesem Zweck wird wiederum das Werkzeugende 178 des Einschraubwerkzeugs 176 mit der Werkzeugaufnahme 40 am Schraubenkopf 30 in Eingriff gebracht und die Knochenschraube 24 so weit in den Wirbelkörper 14 eingeschraubt, bis die Unterseite 44 im Wesentlichen flächig an einer Außenseite des Wirbelkörpers 14 anliegt. Das dornförmige Verankerungselement 174 greift dann in den Wirbelkörper 14 ein und verhindert eine Rotation des Lagerteils 20 um die Längsachse 58.

Falls die verwendeten Lagerteile 20, wie oben beschrieben, zusätzliche Befestigungselementaufnahmen 162 aufweisen, können diese genutzt werden, um die Lagerteile 20 mit zusätzlichen Befestigungselementen 164 gegen ein Verdrehen um die Längsachse 58 zu sichern. Um die Befestigungselemente 164 in definierter Weise einbringen zu können, kann optional ein Ausrichtinstrument 284 von einem Operateur genutzt werden. Es umfasst einen formschlüssig in die Zentrierhülse 96 von proximal her kommend einführbaren Kupplungszapfen 286, welcher über einen in die Längsbohrung 34 eingesetzten Führungsdraht 287 an die Knochenverankerungsvorrichtung heranführbar und über einen Haltekörper 290 mit einer Ausrichthülse 288 gekoppelt ist. Eine Längsachse 292 der Ausrichthülse 288 wird automatisch so positioniert, dass sie mit der Längsachse 172 zusammenfällt. Mittels eines Andorninstruments 294, welches einen langgestreckten Schaft 296 umfasst, an dessen proximalem, Ende ein Griff 298 angeordnet ist mit einer sich quer zur Längsachse 292 erstreckenden Schlagfläche, und ein distales Ende in Form eines angespitzten Dorns 302, wird die Ausrichthülse 288 am Wirbelkörper 14 positioniert. Der Schaft 296 ist mit seinem Außendurchmesser angepasst an den Innendurchmesser der Ausrichthülse 288. Der Dorn 302 kann beispielsweise durch Schlagen mit einem nicht dargestellten Hammer auf die Schlagfläche 300 in den Wirbelkörper 14 eingetrieben werden.

Nach Entfernen des Andorninstruments 294 kann durch die Ausrichthülse 288 das Befestigungselement 164, beispielsweise in Form einer Knochenschraube, eingeführt und mit einem geeigneten Einschraubwerkzeug 304 in den Wirbelkörper 14 eingeschraubt werden. Durch das Andornen des Wirbelkörpers 14 mit dem Dorn 302 wird sichergestellt, dass das Befestigungselement 164 in gewünschter Weise in den Wirbelkörper 14 eingebracht werden kann.

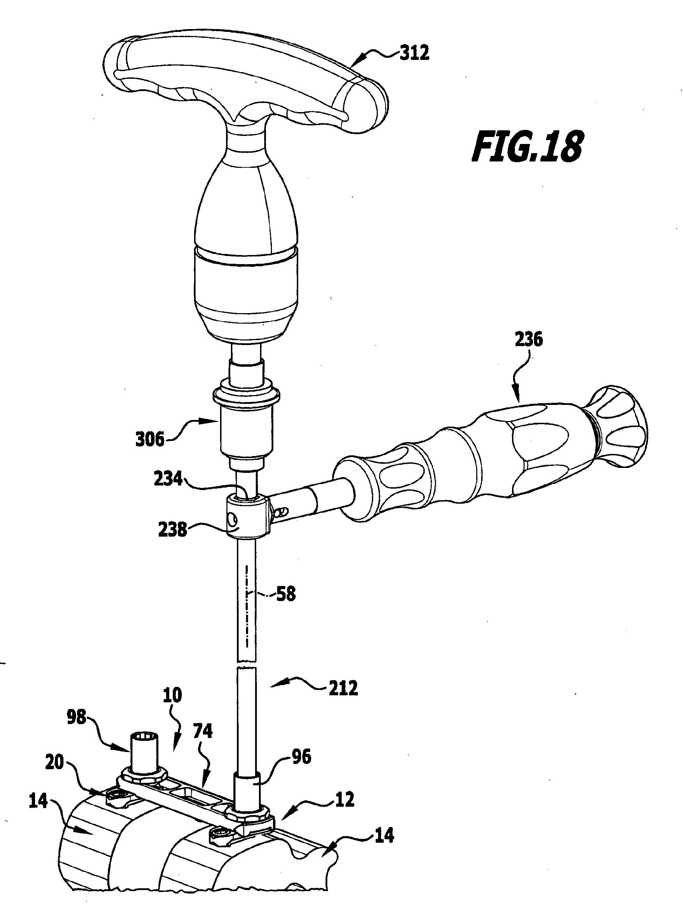

Das Wirbelsäulenfixationssystem 10 ist nun, wie in den Figuren 17 und 18 schematisch dargestellt, an den Wirbelkörpern 14 fixiert. Allerdings sind die Lagerteile 20 und die Verankerungsteile 18 noch nicht gegeneinander gespannt, da die Fixierfläche 124 und der Fixierflächenbereich 146 noch immer beabstandet sind. In einem letzten Schritt ist daher noch das Fixierelement 116 in distaler Richtung gegen den Schraubenkopf 30 zu spannen, und zwar derart, dass die Fixierfläche 124 gegen den Fixierflächenbereich 146 drückt. Zu diesem Zweck wird nochmals das Halteinstrument 212 mit dem Zentrierelement 98 in der bereits oben beschriebenen Weise gekoppelt.

Durch die Führungshülse 214 wird ein Schaft 308 eines weiteren Drehmomentschlüssels-306 eingeführt, welcher an seinem distalen Ende in Form eines Werkzeugendes 310 ausgebildet ist, welches mit der Werkzeugelementaufnahme 128 in Eingriff bringbar ist. Bei dem Drehmomentschlüssel 306 kann es sich insbesondere um das Einschraubwerkzeug 176 handeln. Es sei angemerkt, dass das Werkzeugende 310 ausschließlich mit der Werkzeugelementaufnahme 128 in Eingriff steht, nicht jedoch mit dem proximalen Fixierelementabschnitt 120. Dies bedeutet, dass lediglich Drehmomente auf den distalen Fixierelementabschnitt 118 mittels des Drehmomentschlüssels 306 übertragen werden können. Ein Operateur hält vorzugsweise den Handgriff 236 mit einer Hand und dreht mit seiner anderen Hand an einem T-förmigen Griff 312 des Drehmomentschlüssels 306 im Uhrzeigersinn. Wie bereits oben beschrieben, liegen die Anschlagflächen 102 und 144 aneinander an, so dass durch das Aufbringen eines Drehmoments mittels des Drehmomentschlüssels 306 der Sollbruchbereich 222 sowohl zunehmend auf Zug als auch auf Torsion belastet wird. Wird ein Bruchmoment des Sollbruchbereichs 122 überschritten, wird dieser durch die eingeleiteten Zug- und Torsionskräfte zerstört und der distale Fixierelementabschnitt 118 vom proximalen Fixierelementabschnitt 120 irreversibel getrennt. Mit dem Drehmomentschlüssel 306 kann nun der distale Fixierelementabschnitt 118 weiter in distaler Richtung bewegt werden, bis der Drehmomentschlüssel 306 beim Überschreiten eines voreingestellten Drehmoments auslöst. Das voreingestellte Drehmoment am Drehmomentschlüssel 306 entspricht dem gewünschten Anzugsmoment, mit welchem der distale Fixierelementabschnitt 118 gegen den Schraubenkopf 30 gespannt werden soll. Das Fixierelement 116 beziehungsweise dessen distaler Fixierelementabschnitt 118 bilden somit ein Klemmelement aus zum klemmenden Halten des Schraubenkopfs 30 des Verankerungsteils 18 im Sitz 48 des Lagerteils 20.

Der Drehmomentschlüssel 306 kann nun in proximaler Richtung aus der Führungshülse 214 herausgezogen werden. Das Halteinstrument 212 ist nach wie vor mit dem Zentrierelement 98 gekoppelt. Da der distale Fixierelementabschnitt 118 und der proximale Fixierelementabschnitt 120 voneinander getrennt sind, besteht nunmehr keine feste Verbindung mehr zwischen dem Zentrierelement 98 und dem Lagerteil 20. Das Zentrierelement 98 kann daher mittels des mit ihm gekoppelten Halteinstruments 212 in proximaler Richtung vom Lagerteil 20 abgezogen werden. Der proximale Fixierelementabschnitt 120 ist jedoch in der oben beschriebenen Weise an der Zentrierhülse 96 gehalten und kann nicht aus dieser herausfallen. Er wird gleichzeitig mit dem Entfernen der Zentrierhülse 96 vom Operationsgebiet entfernt.

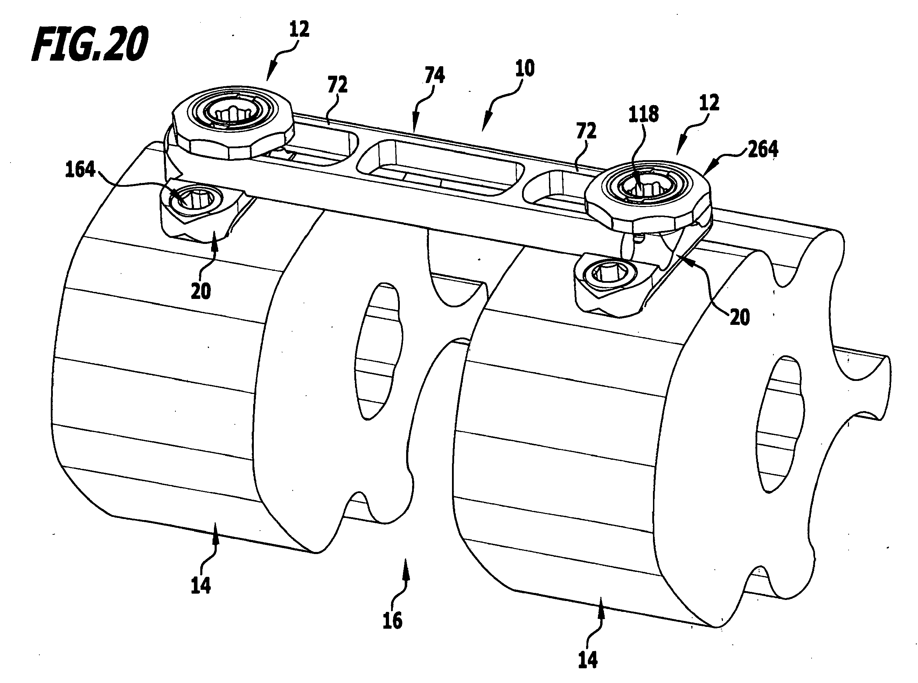

Wie in Figur 20 dargestellt, sind nun die beiden benachbarten Wirbelkörper 14 in gewünschter Weise dauerhaft fest in einem vorgegebenen Abstand voneinander miteinander verbunden.

Das am Lagerteil 20 vormontierte Zentrierelement 98 bildet somit nur temporär einen Teil der Verankerungsvorrichtung 12. Das Fixierelement 116 verbleibt am Ende nur mit seinem distalen Fixierelementabschnitt 118 im Körper des Patienten. Der proximale Fixierelementabschnitt 120 bildet somit zusammen mit der Zentrierhülse 96 einen Teil des beschriebenen Instrumentariums und wird vor dem Verschließen des Körpers des Patienten in der beschriebenen Weise entfernt.

## Patentansprüche

1. Chirurgische Knochenverankerungsvorrichtung (12) für ein Wirbelsäulenfixationssystem (10), umfassend einen Verankerungsteil (18) zum Verankern in oder an einem Knochen (14), einen Lagerteil (20) zum Lagern mindestens eines Verbindungselements (74) zum Festlegen an mindestens zwei Knochenverankerungsvorrichtungen (12) des Wirbelsäulenfixationssystems (10) und ein Fixierelement (116), wobei der Verankerungsteil (18) und der Lagerteil (20) aneinander gelagert, in einer Montagestellung relativ zueinander bewegbar und mit dem Fixierelement (116) in einer Implantationsstellung relativ zueinander festlegbar sind, welches Fixierelement (116) einstückig ausgebildet ist und einen proximalen Fixierelementabschnitt (120), einen distalen Fixierelementabschnitt (118) und einen Sollbruchbereich (122) umfasst, welcher Sollbruchbereich (122) zwischen dem proximalen und dem distalen Fixierelementabschnitt (120, 118) ausgebildet ist, wobei am distalen Fixierelementabschnitt (118) eine Werkzeugelementaufnahme (128) vorgesehen ist zum kraft- und/oder formschlüssigen in Eingriff Bringen mit einem Werkzeug (176) zum Überführen der Knochenverankerungsvorrichtung (12) von der Montagestellung in die Implantationsstellung, **gekennzeichnet durch** ein Zentrierelement (98), welches mit dem Fixierelement (116) am Lagerteil (20) gehalten ist.

2. Chirurgische Knochenverankerungsvorrichtung nach Anspruch 1, **dadurch gekennzeichnet, dass** das Zentrierelement (98) in Form einer Zentrierhülse (96) ausgebildet ist.

3. Chirurgische Knochenverankerungsvorrichtung nach Anspruch 1 oder 2, **gekennzeichnet durch** eine Positioniereinrichtung (88) zum Positionieren des Zentrierelements (98) und des Lagerteils (20) relativ zueinander in der Montagestellung.

4. Chirurgische Knochenverankerungsvorrichtung nach einem der voranstehenden Ansprüche, **dadurch gekennzeichnet, dass** das Fixierelement (116) das Zentrierelement (98) in der Montagestellung kraft- und/oder formschlüssig am Lagerteil (20) hält.

5. Chirurgische Knochenverankerungsvorrichtung nach einem der Ansprüche 2 bis 4, **gekennzeichnet durch** eine Kopplungseinrichtung (150) zum Koppeln des Zentrierelements (98) und des Fixierelements (116).

6. Chirurgische Knochenverankerungsvorrichtung nach Anspruch 5, **dadurch gekennzeichnet, dass** die Positioniereinrichtung (88) und die Kopplungseinrichtung (150) das Zentrierelement (98) in der Montagestellung und in der Kopplungsstellung um eine vom Zentrierelement (98) definierte Längsachse (52) unverdrehbar am Lagerteil (20) sichern.

7. Chirurgische Knochenverankerungsvorrichtung nach Anspruch 5 oder 6, **dadurch gekennzeichnet, dass** das Fixierelement (116) eine Fixierfläche (124) aufweist, welche in der Implantationsstellung an einem Fixierflächenbereich (146) des Verankerungsteils (18) anliegt, und dass die Fixierfläche (124) vom Fixierflächenbereich (146) beabstandet ist, wenn die Kopplungseinrichtung (150) die Kopplungsstellung einnimmt und solange der distale und der proximale Fixierelementabschnitt (118, 120) über den Sollbruchbereich (122) miteinander verbunden sind.

8. Chirurgische Knochenverankerungsvorrichtung nach einem der voranstehenden Ansprüche, **dadurch gekennzeichnet, dass** der Lagerteil (20) und der Verankerungsteil (18) relativ zueinander beweglich sind, wenn der Sollbruchbereich (122) unbeschädigt ist.

9. Chirurgische Knochenverankerungsvorrichtung nach einem der voranstehenden Ansprüche, **dadurch gekennzeichnet, dass** der distale und der proximale Fixierelementabschnitt (118, 120) irreversibel voneinander trennbar sind durch Zerstören des Sollbruchbereichs (122).

10. Chirurgische Knochenverankerungsvorrichtung nach einem der voranstehenden Ansprüche, **dadurch gekennzeichnet, dass** der Sollbruchbereich (122) durch eine Schwächung des Fixierelements (116) zwischen dem distalen und dem proximalen Fixierelementabschnitt (118, 120) ausgebildet ist.

11. Chirurgische Knochenverankerungsvorrichtung nach einem der voranstehenden Ansprüche, **dadurch gekennzeichnet, dass** der proximale Fixierelementabschnitt (120) einen größeren Außendurchmesser aufweist als der distale Fixierelementabschnitt (118).

12. Chirurgische Knochenverankerungsvorrichtung nach einem der Ansprüche 2 bis 11, **gekennzeichnet durch** eine Sicherungseinrichtung (156) zum Sichern des proximalen Fixierelementabschnitts (120) am Zentrierelement (98) nach dem Trennen der distalen und proximalen Fixierelementabschnitte (118, 120) voneinander.

13. Chirurgische Knochenverankerungsvorrichtung nach Anspruch 12, **dadurch gekennzeichnet, dass** die Sicherungseinrichtung (156) eine Innenverzahnung (110) am Zentrierelement (98) proximalseitig des am Zentrierelement (98) vorgesehenen Kopplungsglieds (152) und eine korrespondierende Außenverzahnung (140) des proximalen Fixierelementabschnitts (120) umfasst.

14. Wirbelsäulenfixationssystem (10) umfassend mindestens eine chirurgische Knochenverankerungsvorrichtung (12) und mindestens ein Verbindungselement (74) zum Festlegen an mindestens zwei Knochenverankerungsvorrichtungen (12) des Wirbelsäulenfixationssystems (10), welche mindestens eine chirurgische Knochenverankerungsvorrichtung (12) einen Verankerungsteil (18) zum Verankern in oder an einem Knochen (14), einen Lagerteil (20) zum Lagern mindestens eines Verbindungselements (74) zum Festlegen an mindestens zwei Knochenverankerungsvorrichtungen (12) des Wirbelsäulenfixationssystems (10) und ein Fixierelement (116) umfasst, wobei der Verankerungsteil (18) und der Lagerteil (20) aneinander gelagert, in einer Montagestellung relativ zueinander bewegbar und mit dem Fixierelement (116) in einer Implantationsstellung relativ zueinander festlegbar sind, welches Fixierelement (116) einstückig ausgebildet ist und einen proximalen Fixierelementabschnitt (120), einen distalen Fixierelementabschnitt (118) und einen Sollbruchbereich (122) umfasst, welcher Sollbruchbereich (122) zwischen dem proximalen und dem distalen Fixierelementabschnitt (120, 118) ausgebildet ist, wobei am distalen Fixierelementabschnitt (118) eine Werkzeugelementaufnahme (128) vorgesehen ist zum kraftund/oder formschlüssigen in Eingriff Bringen mit einem Werkzeug (176) zum Überführen der Knochenverankerungsvorrichtung (12) von der Montagestellung in die Implantationsstellung, **gekennzeichnet durch** ein Zentrierelement (98), welches mit dem Fixierelement (116) am Lagerteil (20) gehalten ist.

15. Wirbelsäulenfixationssystem nach Anspruch 14, **gekennzeichnet durch** eine Knochenverankerungsvorrichtung (12) nach einem der Ansprüche 2 bis 13.

## Claims

1. Surgical bone anchoring device (12) for a spinal column fixation system (11), comprising an anchoring part (18) adapted for anchoring in or on a bone (14), a bearing part (20) adapted for mounting of at least one connection element (74) thereon, said connection element (74) being adapted for fixing to at least two bone anchoring devices (12) of the spinal column fixation system (10), and a fixing element (116), wherein said anchoring part (18) and said bearing part (20) are mounted on each other, movable relative to each other in an assembly position and fixable relative to each other by the fixing element (116) in an implantation position, said fixing element (116) being of integral construction and having a proximal fixing element section (120), a distal fixing element section (118) and a predetermined break-off area (122), said predetermined break-off area (122) being formed between the proximal and distal fixing element sections (120, 118), wherein the distal fixing element section (188) having provided thereon a tool element receptacle (128) for engagement by force locking and/or positive locking with a tool (176) for transferring the bone anchoring device (12) from the assembly position to the implantation position, **characterized by** a centering element (98), which is held by the fixing element (116) on the bearing part (20).

2. Surgical bone anchoring device in accordance with claim 1, **characterized in that** the centering element (98) is in the form of a centering sleeve (96).

3. Surgical bone anchoring device in accordance with claim 1 or 2, **characterized by** a positioning device (88) for positioning the centering element (98) and the bearing part (20) relative to each other in the assembly position.

4. Surgical bone anchoring device in accordance with any one of the preceding claims, **characterized in that** the fixing element (116) holds the centering element (98) by force locking and/or positive locking on the bearing part (20) in the assembly position.

5. Surgical bone anchoring device in accordance with any one of claims 2 to 4, **characterized by** a coupling device (150) for coupling the centering element (98) and the fixing element (116).

6. Surgical bone anchoring device in accordance with claim 5, **characterized in that** the positioning device (88) and the coupling device (150) secure the centering element (98) in the assembly position and in the coupling position on the bearing part (20) so that the centering element (98) is unable to rotate about a longitudinal axis (52) defined by the centering element (98).

7. Surgical bone anchoring device in accordance with claim 5 or 6, **characterized in that** the fixing element (116) has a fixing surface (124) which, in the implantation position, bears on a fixing surface area (146) of the anchoring part (18), and **in that** the fixing surface (124) is spaced from the fixing surface area (145) when the coupling device (150) assumes the coupling position and so long as the distal and proximal fixing element sections (118, 120) are connected to each other via the predetermined break-off area (122).

8. Surgical bone anchoring device in accordance with any one of the preceding claims, **characterized in that** the bearing part (20) and the anchoring part (18) are movable relative to each other when the predetermined break-off area (122) is undamaged.

9. Surgical bone anchoring device in accordance with any one of the preceding claims, **characterized in that** the distal and proximal fixing element sections (188, 120) are irreversibly separable from each other by destroying the predetermined break-off area (122).

10. Surgical bone anchoring device in accordance with any one of the preceding claims, **characterized in that** the predetermined break-off area (122) is formed by a weakening of the fixing element (116) between the distal and proximal fixing element sections (118, 120).

11. Surgical bone anchoring device in accordance with any one of the preceding claims, **characterized in that** the proximal fixing element section (120) has a larger outer diameter than the distal fixing element section (118).

12. Surgical bone anchoring device in accordance with any one of claims 2 to 11, **characterized by** a securing device (156) for securing the proximal fixing element section (120) on the centering element (98) after separation of the distal and proximal fixing element sections (118, 120) from each other.

13. Surgical bone anchoring device in accordance with claim 12, **characterized in that** the securing device (156) comprises an internal toothing (110) on the centering element (98) proximally of the coupling member (152) provided on the centering element (98), and a corresponding external toothing (140) of the proximal fixing element section (120).

14. Spinal column fixation system (10) comprising at least one surgical bone anchoring device (12) and at least one connection element (74) for fixing to at least two bone anchoring devices (12) of the spinal column fixation system (10), said at least one surgical bone anchoring device (12) comprising an anchoring part (18) adapted for anchoring in or on a bone (14), a bearing part (20) adapted for mounting of at least one connection element (74) thereon, the connection element (74) being adapted for fixing to at least two bone anchoring devices (12) of the spinal column fixation system (10), and a fixing element (116), wherein said anchoring part (18) and said bearing part (20) are mounted on each other, movable relative to each other in an assembly position and fixable relative to each other by the fixing element (116) in an implantation position, said fixing element (116) being of integral construction and having a proximal fixing element section (120), a distal fixing element section (118) and a predetermined break-off area (122), said predetermined break-off area (122) being formed between the proximal and distal fixing element sections (120, 118), wherein the distal fixing element section (118) having provided thereon a tool element receptacle (128) for engagement by force locking and/or positive locking with a tool (176) for transferring the bone anchoring device (12) from the assembly position to the implantation position, **characterized by** a centering element (98), which is held by the fixing element (116) on the bearing part (20).

15. Spinal column fixation system in accordance with claim 14, **characterized by** a bone anchoring device (12) in accordance with any one of claims 2 to 13.

## Revendications

1. Dispositif chirurgical d'ancrage dans l'os (12) pour un système de fixation (10) de colonne vertébrale, comprenant une pièce d'ancrage (18) pour l'ancrage dans ou sur un os (14), une pièce de palier de support (20) pour le montage d'au moins un élément de liaison (74) destiné à être fixé à au moins deux dispositifs d'ancrage dans l'os (12) du système de fixation de colonne vertébrale (10), et un élément de fixation (116), dispositif d'ancrage dans lequel la pièce d'ancrage (18) et la pièce de palier de support (20) montées l'une contre l'autre, peuvent être déplacées l'une par rapport à l'autre dans une position de montage, et peuvent être immobilisées l'une par rapport à l'autre avec l'élément de fixation (116) dans une position d'implantation, cet élément de fixation (116) étant réalisé d'un seul tenant et comprenant un tronçon d'élément de fixation proximal (120), un tronçon d'élément de fixation distal (118) et une zone de rupture programmée (122), cette zone de rupture programmée (122) étant réalisée entre le tronçon d'élément de fixation proximal et celui distal (120, 118), et dans lequel sur le tronçon d'élément de fixation distal (118) est prévu un logement d'élément d'outil (128) pour l'amenée en prise, par adhérence et/ou complémentarité de formes, avec un outil (176), en vue de transférer le dispositif d'ancrage dans l'os (12) de la position de montage dans la position d'implantation,
**caractérisé par** un élément de centrage (98), qui est maintenu avec l'élément de fixation (116) sur la pièce de palier de support (20).

2. Dispositif chirurgical d'ancrage dans l'os selon la revendication 1, **caractérisé en ce que** l'élément de centrage (98) est réalisé sous la forme d'une douille de centrage (96).

3. Dispositif chirurgical d'ancrage dans l'os selon la revendication 1 ou la revendication 2, **caractérisé par** un dispositif de positionnement (88) pour positionner l'élément de centrage (98) et la pièce de palier de support (20) relativement l'une par rapport à l'autre dans la position de montage.

4. Dispositif chirurgical d'ancrage dans l'os selon l'une des revendications précédentes, **caractérisé en ce que** l'élément de fixation (116) maintient, par adhérence et/ou complémentarité de formes, l'élément de centrage (98) sur la pièce de palier de support (20), dans la position de montage.

5. Dispositif chirurgical d'ancrage dans l'os selon l'une des revendications 2 à 4, **caractérisé par** un dispositif de couplage (150) pour le couplage de l'élément de centrage (98) et de l'élément de fixation (116).

6. Dispositif chirurgical d'ancrage dans l'os selon la revendication 5, **caractérisé en ce que** le dispositif de positionnement (88) et le dispositif de couplage (150) sécurisent, dans la position de montage et dans la position de couplage, l'élément de centrage (98) sur la pièce de palier de support (20), de manière non tournante autour d'un axe longitudinal (52) défini par l'élément de centrage (98).

7. Dispositif chirurgical d'ancrage dans l'os selon la revendication 5 ou la revendication 6, **caractérisé en ce que** l'élément de fixation (116) présente une surface de fixation (124), qui, dans la position d'implantation, s'appuie sur une zone de surface de fixation (146) de la pièce d'ancrage (18), et **en ce que** la surface de fixation (124) est espacée de la zone de surface de fixation (146), lorsque le dispositif de couplage (150) prend la position de couplage et aussi longtemps que le tronçon d'élément de fixation distal et celui proximal (118, 120) sont reliés par l'intermédiaire de la zone de rupture programmée (122).

8. Dispositif chirurgical d'ancrage dans l'os selon l'une des revendications précédentes, **caractérisé en ce que** la pièce de palier de support (20) et la pièce d'ancrage (18) sont mobiles relativement l'une par rapport à l'autre, lorsque la zone de rupture programmée (122) n'est pas détruite.

9. Dispositif chirurgical d'ancrage dans l'os selon l'une des revendications précédentes, **caractérisé en ce que** le tronçon d'élément de fixation distal et celui proximal (118, 120) peuvent être séparés l'un de l'autre de manière irréversible, par destruction de la zone de rupture programmée (122).

10. Dispositif chirurgical d'ancrage dans l'os selon l'une des revendications précédentes, **caractérisé en ce que** la zone de rupture programmée (122) est formée par un affaiblissement de l'élément de fixation (116) entre le tronçon d'élément de fixation distal et celui proximal (118, 120).

11. Dispositif chirurgical d'ancrage dans l'os selon l'une des revendications précédentes, **caractérisé en ce que** le tronçon d'élément de fixation proximal (120) présente un diamètre extérieur plus grand que celui du tronçon d'élément de fixation distal (118).

12. Dispositif chirurgical d'ancrage dans l'os selon l'une des revendications 2 à 11, **caractérisé par** un dispositif de sécurisation (156) pour sécuriser le tronçon d'élément de fixation proximal (120) sur l'élément de centrage (98) après la séparation des tronçons d'élément de fixation distal et proximal (118, 120) l'un de l'autre.

13. Dispositif chirurgical d'ancrage dans l'os selon la revendication 12, **caractérisé en ce que** le dispositif de sécurisation (156) comprend une denture intérieure (110) sur l'élément de centrage (98), du côté proximal de l'organe de couplage (152) prévu sur l'élément de centrage (98), et une denture extérieure (140) correspondante du tronçon d'élément de fixation proximal (120).

14. Système de fixation de colonne vertébrale (10) comprenant au moins un dispositif chirurgical d'ancrage dans l'os (12) et au moins un élément de liaison (74) destiné à être fixé à au moins deux dispositifs d'ancrage dans l'os (12) du système de fixation de colonne vertébrale (10), ledit au moins un dispositif chirurgical d'ancrage dans l'os (12) comprenant une pièce d'ancrage (18) pour l'ancrage dans ou sur un os (14), une pièce de palier de support (20) pour le montage d'au moins un élément de liaison (74) destiné à être fixé à au moins deux dispositifs d'ancrage dans l'os (12) du système de fixation de colonne vertébrale (10), et un élément de fixation (116), dispositif d'ancrage dans lequel la pièce d'ancrage (18) et la pièce de palier de support (20) montées l'une contre l'autre, peuvent être déplacées l'une par rapport à l'autre dans une position de montage, et peuvent être immobilisées l'une par rapport à l'autre avec l'élément de fixation (116) dans une position d'implantation, cet élément de fixation (116) étant réalisé d'un seul tenant et comprenant un tronçon d'élément de fixation proximal (120), un tronçon d'élément de fixation distal (118) et une zone de rupture programmée (122), cette zone de rupture programmée (122) étant réalisée entre le tronçon d'élément de fixation proximal et celui distal (120, 118), et dans lequel sur le tronçon d'élément de fixation distal (118) est prévu un logement d'élément d'outil (128) pour l'amenée en prise, par adhérence et/ou complémentarité de formes, avec un outil (176), en vue de transférer le dispositif d'ancrage dans l'os (12) de la position de montage dans la position d'implantation,
**caractérisé par** un élément de centrage (98), qui est maintenu avec l'élément de fixation (116) sur la pièce de palier de support (20).

15. Système de fixation de colonne vertébrale selon la revendication 14, **caractérisé par** un dispositif chirurgical d'ancrage dans l'os (12) selon l'une des revendications 2 à 13.
